# EUROPEAN PATENT SPECIFICATION

(11) **EP 3 194 565 B1**
(45) Date of publication and mention of the grant of the patent: **28.11.2018**
(21) Application number: 15775091.0
(22) Date of filing: 29.07.2015
(51) Int. Cl.: C12N 1/18, A21D 8/04, C12R 1/865

(54) **STRAIN OF THE YEAST SPECIES SACCHAROMYCES CEREVISIAE, STRAINS ESSENTIALLY DERIVED FROM IT AND USE THEREOF**
STAMM DER HEFESPEZIES SACCHAROMYCES CEREVISIAE, IM WESENTLICHEN DARAUS ABGELEITETE STÄMME UND VERWENDUNG DAVON
SOUCHE DE L'ESPÈCE DE LEVURE SACCHAROMYCES CEREVISAE, SOUCHES ESSENTIELLEMENT DÉRIVÉES DE CELLE-CI ET LEUR UTILISATION

(30) Priority: 29.07.2014 IT MI20141366
(43) Date of publication of application: 26.07.2017
(73) Proprietor: La Pizza + 1 S.p.A., 29027 Podenzano (IT)
(72) Inventor: SANTE, Ludovico, I-29019 San Giorgio Piacentino (IT)
(74) Representative: Zaccaro, Elisabetta
(86) International application number: PCT/EP2015/067431
(87) International publication number: WO 2016/016334

(56) References cited:
- US-A- 5 480 798
- US-A- 5 776 526
- "Wheat Beer", Shrivers Beer & Wine Supplies , 22 March 2010 (2010-03-22), XP002751194, Retrieved from the Internet: URL:http://shriversbeerwinesupply.com/prod uct_info.php?products_id=177?osCsid=nq1pcm uua5ajfrkaeeis4vf1o0 [retrieved on 2015-11-20]

## Description

### Field of the invention

The present invention relates to a yeast strain of the species *Saccharomyces cerevisiae* isolated from spontaneously leavened dough, which is able to remain viable at temperatures below 8°C and inactive for carbon dioxide production, and is able to restore normal fermenting and leavening capacity when it is shifted to the optimal temperature range. Furthermore, the invention relates to solid and fluid doughs, comprising at least water, flour and yeast according to the invention, to the uses of such doughs and their production.

### State of the art

The species *Saccharomyces cerevisiae* (S.cerevisiae) Meyen ex E.C. Hansen (1883) is the *type* species of the genus *Saccharomyces,* represented by the *type strain Saccharomyces cerevisiae* CBS 1171^{T} (Vaughan-Martini & Martini, 2011). It is a yeast species defined as domesticated, its ideal environment being industrial processes involving fermentation of sugar-rich substrates such as grape juice, malt beer, flour dough for bakery and confectionery. Morphologically it is characterized by ellipsoidal cells that in the asexual state reproduce by multilateral budding and rarely form possibly non-septate pseudohyphae (Vaughan-Martini & Martini, 2011). Strains of *Saccharomyces cerevisiae* species can be haploid although often they are found in a diploid or higher ploidy state. Aneuploid strains are not uncommon even for strains of technological interest with applications in winemaking, brewing, baking (Legras et al., 2007). In their sexual reproduction, conjugation takes place between cells of different *mating* type. More commonly, cells have ploidy equal to or greater than two and directly convert to asci. In fact conjugation events are considered rare for the species *Saccharomyces cerevisiae* known to occur at very low concentration in natural environments; although cell concentration increases during the fermentation processes performed by man, the elevated concentration of sugar represses conjugation due to the so-called Crabtree effect. Asci contain one to four ascospores of spherical or short ellipsoidal shape (Vaughan-Martini & Martini, 2011).

Fermentation is normally vigorous for the species *Saccharomyces cerevisiae.* This species ferments glucose, sucrose, maltose and raffinose. It does not ferment lactose, trehalose and melibiose. It ferments galactose variably. Growth on nitrate as the only nitrogen source has not been observed. Starch or similar compounds are not produced. Optimal growth temperatures are comprised between 25°C and 30°C, whereas only some strains can grow at 37°C or 40°C and few of those represent a possible risk for human health as they become opportunistic pathogens in immune deficient individuals (Vaughan-Martini & Martini, 2011; Thygesen et al., 2012).

Concerning the genome, its G+C content (% G + C) is comprised between 39% and 41%. The genome consists of 16 chromosomes. Available genome sizes can vary from 10.26 Mbp for *Saccharomyces cerevisiae* CLIB382 strain (GeneBank Assembly ID: GCA_000209345.1) to 14.27 Mbps for *Saccharomyces cerevisiae* Y10 strain (GeneBank Assembly ID: GCA_000192375.1). The mitochondrial chromosome is approximately 0.086 Mb (Foury et al., 1998), although strain-dependent variations in size and sequence of this important genetic element have been reported (Raiser et al., 2012). Extrachromosomal genetic elements can be present, such as plasmids, the best known and best studied being the 2µm plasmid used as the basis for developing genetic vectors in order to engineer the strains of interest (Karim et al., 2013).

The studies on various strains of *Saccharomyces cerevisiae* have mainly focused on the field of bakery products, seeking strains of yeasts that would allow more than one week-long preservation of dough made of flour, water and yeast. In fact, these products made with "known" yeasts can be stored at refrigerated temperatures (4-8°C) only for few days. This is because "known" yeasts are insensitive to such temperatures and continue their fermenting activity, thereby producing carbon dioxide and ethanol as final metabolites. After 3 days of storage at refrigerated temperature, the continuous build-up of carbon dioxide inside the dough packages causes an unacceptable swelling from the standpoint of product quality. Moreover, the metabolic activity of yeast continues, thus impacting negatively on the organoleptic and rheological properties of dough. In particular, once the package is opened, the dough is characterized by unpleasant odours due to a high ethanol content.

In order to overcome this problem, sealed packages with an escape gas valve for surplus gas have been tried over the years. However, the top producers of raw dough have gradually abandoned this type of package because of three issues: 1) the cost of valve is too high compared to the cost of the package and the small value of the product content; 2) under conditions where the appropriate amount of yeast is used for good leavening, anyway the valve does not let the internal and external package pressures to become equal. It is therefore necessary to dose very low amounts of yeast even when using the valve, resulting in poorly developed and structured dough; 3) even in presence of the valve and a reduced pressure inside the package, the metabolic activity of yeast is not maintained stable, thus compromising within few days the rheological and organoleptic properties of the dough, resulting in a weakened structure of the dough (making it impossible to roll out thereafter) and in strongly altered odour and taste. For all these reasons, strains of *Saccharomyces cerevisiae* have been sought which can remain inactive but viable for long time at temperatures ranging from 0°C to 8°C and resume normal metabolic activity, and consequent production of carbon dioxide necessary for leavening, once they are returned to optimal growth temperatures. This characteristic is also called "*Low Temperature inactivation*", abbreviated as LTi. US5480798 and US5776526 describe yeast strains of the *Saccharomyces cerevisiae* species characterized by the LTi phenotype, suggested to be applied in dough leavening . These strains, namely *S. cerevisiae* NCIMB 40328; *S*. *cerevisiae* NCIMB 40329; *S. cerevisiae* NCIMB 40330; *S. cerevisiae* NCIMB 40331; *S. cerevisiae* NCIMB 40332; *S. cerevisiae* NCIMB 40611 ; *S. cerevisiae* NCIMB 40612; *S. cerevisiae* NCIMB 40613; *S. cerevisiae* NCIMB 40614 and *S. cerevisiae* NCIMB 40615 were obtained through *in-vitro* techniques and subsequent selection. Therefore, the aim of the present invention is to provide a yeast strain of the species *Saccharomyces cerevisiae,* devoid of the aforementioned disadvantages of the known strains already in use for the same application.

### Summary of the invention

The invention relates therefore to a yeast strain of the species *Saccharomyces cerevisiae* deposited with accession number (or filing number) DSM 29004 at the International Deposit Authority Leibniz-Institut DSMZ - Deutsche Sammlung von Mikroorganismen und Zellkulturen GmbH.

Such yeast strain according to the present invention, isolated from spontaneous leavening dough, can remain viable at temperatures below 8 °C, in presence of water activity less than or equal to 0.98, in an inactive state with respect to carbon dioxide production, and can restore normal fermenting and leavening capacity when it is returned to an optimal temperature range.

In another aspect the invention therefore relates to a solid or fluid dough comprising at least water, flour and yeast of the species *Saccharomyces cerevisiae* deposited with the accession number DSM 29004.

According to yet another aspect, the invention relates to the use of the yeast strain of the species *Saccharomyces cerevisiae,* deposited with accession number DSM 29004 at the International Deposit Authority Leibniz-Institut DSMZ - Deutsche Sammlung von Mikroorganismen und Zellkulturen GmbH, for production of solid or fluid doughs.

According to yet another aspect, the invention relates to a process for preparation of a refrigerated dough in solid or fluid form, prepared by mixing at least water, flour and yeast of the species *Saccharomyces cerevisiae* deposited with the accession number DSM 29004 at the International Deposit Authority Leibniz-Institut DSMZ - Deutsche Sammlung von Mikroorganismen und Zellkulturen GmbH, wherein said dough is packaged in a container.

Therefore the yeast strain of *Saccharomyces cerevisiae* according to the present invention remains surprisingly inactive, although viable for a long time, at temperatures below 8°C and resumes normal metabolic activity producing carbon dioxide enabling leavening, when it is returned to temperatures optimal for growth, and in this its behaviour is dramatically distinct from "known" brewer's yeasts characterized by the aforementioned behaviour and drawbacks with respect to storage of raw doughs.

In a still further embodiment the invention relates to a process for the preparation of a medium having a high alpha-amino nitrogen concentration, comprising the steps of:
a. growing the yeast strain DSM 29004 in a barley malt and wheat malt extract medium for at least 36 hours;
b. separating the yeast from the medium, preferably by filtration or by centrifugation;
c. obtaining a medium having a high alpha- amino nitrogen concentration higher than 300 mg/l, wherein the alpha-amino nitrogen is not added to the medium but results from the yeast growth.

Definitions in the present invention:
- "Strain DSM 29004" is meant to include a yeast strain of the species *Saccharomyces cerevisiae,* filed on 01.07.2014 with accession number (or filing number) DSM 29004 at the International Deposit Authority Leibniz-Institut DSMZ - Deutsche Sammlung von Mikroorganismen und Zellkulturen GmbH;
- "genetic manipulation" is meant to include any technical intervention aimed at directing the process of acquisition of specific genetic characteristics which are expressed as corresponding phenotypic traits, where the technical interventions comprise (Sturley & Young, 1986): (i) crossing natural strains, followed by selection; (ii) production of hybrids followed by selection; (iii) transformation, i.e. insertion of exogenous DNA in chromosomes or in the mitochondrial genome, or other genetic elements such as plasmids. (iv) Induced random mutagenesis usually followed by selection and/or production of hybrids (Nevoigt 2008) can be added as further mode of manipulation;
- "essentially derived variant" is meant to include a variant of the yeast strain of the species *Saccharomyces cerevisiae,* deposited at the DSMZ on 01.07.2014 with accession number DSM 29004, which can be tracked back to it by microsatellite DNA profiling or because of a distinctive genetic trait detectable by genome sequencing and comparative analysis. In the present case, microsatellite DNA profiles of the essentially derived variants may be identical to DSM 29004, but may have sequences different from it in genomic regions other than microsatellite DNA loci as result of spontaneous mutation, induced mutation, hybridization or other genetic manipulation methods; One possible embodiment of any essentially derived variant of the strain DSM 29004 is a lineage of the strain DSM 29004 deposited at the DSMZ on 01.07.2014 with accession number DSM 29004 which shares with it the same microsatellite DNA profiles for 10 loci, as reported in Table 3, but is characterized by an increased osmotic stress tolerance and a greater capacity to survive after drying during the production of the strain DSM 29004 as active dry yeast. The genome sequence of this new lineage essentially derived from the strain DSM 29004 could be characterized by a genome sequence different from the strain DSM 29004 up to 30%, except for the DNA microsatellite loci.
- "Strain of yeast inactive but viable" is meant to include a yeast strain that does not produce carbon dioxide or produces almost imperceptible amounts of it, but is able to resume metabolic activity and to sustain the process of leavening of a dough based on flour and water, increasing the volume up to 3-fold the original volume upon a shift to the optimal growth temperature;
- "Fermentative vigour" refers to the activity of a yeast strain to consume the available sugars and produce carbon dioxide, thereby allowing leavening with a desired texture of the fermented dough;
- "Baked goods" is meant to include bread, pizza, flat bread (focaccia), small savoury scones (tigelle), but also leavened products suitable for frying as fried puffy dough rectangles (gnocco fritto) and fried dumplings (panzerotti);
- "Modified atmosphere" or "protective atmosphere" refers to an atmosphere with gas mixture including carbon dioxide in percentages from 0% to 60% and nitrogen from 0% to 80%, preferably 50% carbon dioxide and 50% nitrogen;
- "Substantial absence of carbon dioxide production" may occur when yeast is added to a dough made of flour and water. Said mixture maintained between 0°C and 8°C results in a possible increase of the carbon dioxide concentration into the package comprised from 0% to 5% of the initial concentration just after sealing the package. Swelling of sealed package could be an indirect sign that gas production by yeast is over the setted range.
- "growth medium" (synonyms: growth medium / cultivation broth/ growth broth) refers to a substrate containing all the compounds (factors) required by a microorganism for the cell replication resulting in the increasing of the single cells number and growth of the population. In this context the microorganisms are intended mainly as yeasts of the species *Saccharomyces cerevisiae* and lactic acid bacteria belonging to the genera *Lactobacillus*, *Pediococcus* and *Leuconostoc.* The factors required by the microorganisms for their growth in the medium belong mainly to the categories: carbon source, assimilable nitrogen source (constituted both by ammonia and free amino acids, the last also known as FAN), vitamins and salts (trace elements). Typical carbon sources are sugarcane molasses, beet molasses, barley malt extract and wheat malt extract;
- "discarded medium" refers to one of the component obtained after separation and harvest of the viable yeast cells from the growth medium, that is characterized by a lower concentration in carbon source and assimilable nitrogen because they are converted in cell mass during the growth of the yeasts, but it still contains FAN and other essential factors, also derived from the metabolism of the cultivated yeasts;
- not vital cell/ not viable cells, cell extracts, cell lysate" refers to by-products derived from the growth of the strain DSM 29004 in a culture medium. Cell debris derived from not viable cells, cell extracts and cell lysate can therefore be separated from viable cell by centrifugation and/or filtration, then recovered in the discarded medium. The composition of not vital cell/ not viable cells, cell extracts, cell lysate is strictly linked to the unique metabolic and physiological properties of the DSM 29004 strain.

### DESCRIPTION OF THE FIGURES

The invention will now be described in detail and in reference to the attached Figures, wherein:

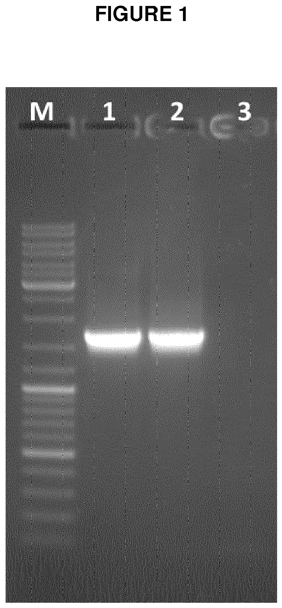

**Figure 1** shows the picture of an agarose gel displaying the results of species-specific PCR for *Saccharomyces cerevisiae,* thus providing confirmation that DSM 29004 strain belongs to this species, as described in Example 1. The loading order is as follows: M) size marker O'GeneRuler DNA Ladder Mix, Ready-to-Use 100 to 10.000 bp (Thermo Scientific); 1) *Saccharomyces cerevisiae* DSM 29004; 2) *Saccharomyces cerevisiae* CBS 1171^{T}; 3) No Template Control;

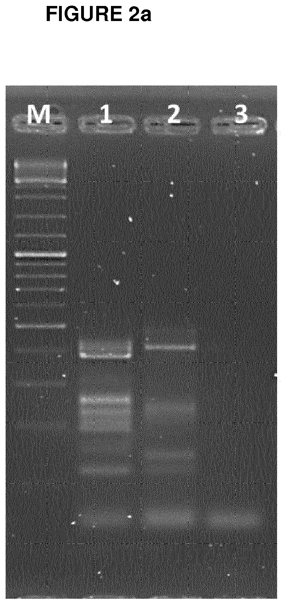

**Figure 2a** shows, in a reproduction of the picture of an agarose gel, PCR results displaying the profile of microsatellite DNA for SC8132X, YOR267C SCPTSY7 *loci* and for the C5 *locus* after electrophoresis on 3% w/v agarose gel, like in Example 1. The loading order is as follows: M) size marker O'GeneRuler DNA Ladder Mix, Ready-to-Use 100 to 10.000 bp (Thermo Scientific); 1) *Saccharomyces cerevisiae* DSM 29004; 2) *Saccharomyces cerevisiae* CBS 1171T; 3) No Template Control;

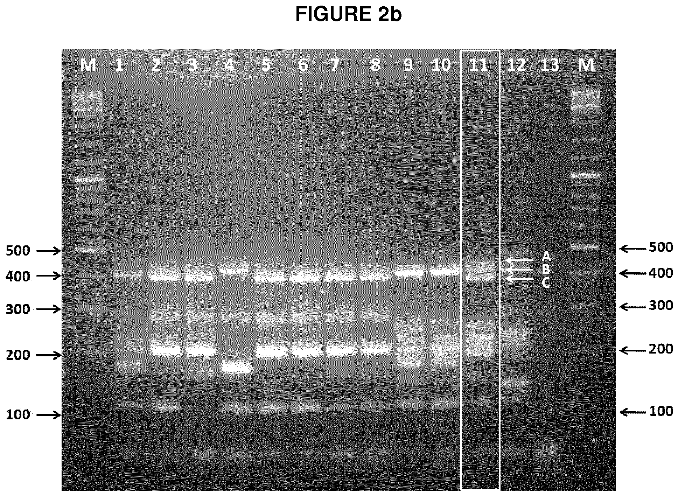

**Figure 2b** shows the photo of an agarose gel with the PCR results displaying the DNA microsatellite profile for the SC8132X, YOR267C SCPTSY7 *loci* and for the C5 *locus* after electrophoresis on 3% w/v agarose gel, as indicated in Example 8. The loading order is as follows: M) size marker O'GeneRuler DNA Ladder Mix, Ready-to-Use 100 to 10.000 bp (Thermo Scientific); 1) *Saccharomyces cerevisiae* NCIMB 40611 ; 2) *S. cerevisiae* NCIMB 40612; 3) *S. cerevisiae* NCIMB 40613; 4) *S. cerevisiae* NCIMB 40614; 5) *S. cerevisiae* NCIMB 40615; 6) S. *cerevisiae* NCIMB 40328; 7) *S. cerevisiae* NCIMB 40329; 8) *S. cerevisiae* NCIMB 40330; 9) *S. cerevisiae* NCIMB 40331; 10) S. *cerevisiae* NCIMB 40332; 11) *S. cerevisiae* DSM 29004; 12) *S. cerevisiae* CBS 1171^{T}; 13) No Template Control.

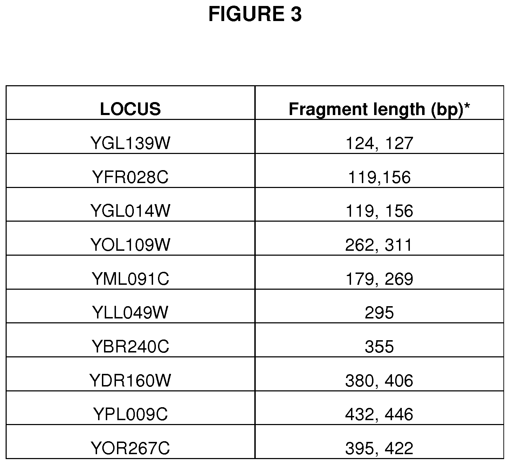

**Figure 3** shows microsatellite DNA profiles for YGL139W, YFR028C, YGL014W, YOL109W, YML091C, YLL049W, YBR240C, YDR160W, YPL009C, YOR267C *loci* by separation of fragments in a capillary as detailed in Example 1. Sizes (*) are expressed as base pairs (bp) and with +/-1 bp error. The main peaks observed for each *locus,* up to a maximum of two, are reported;

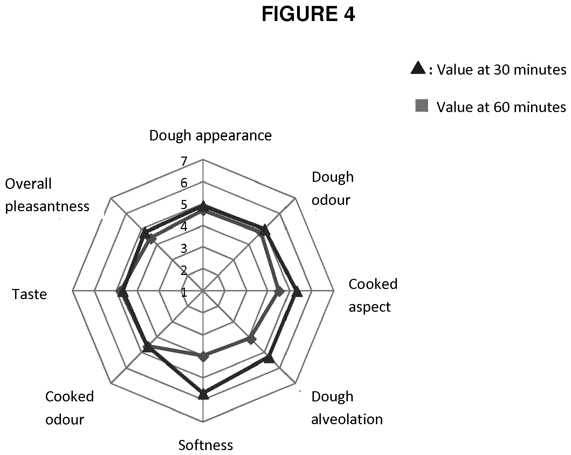

**Figure 4** shows data of the test panel displayed in a Kiviat diagram or "radar chart" reporting the data of multiple variables: dough appearance, odor of the dough, appearance of the cooked product, dough alveolation, softness, odor of the cooked product, overall taste and pleasantness, as described in Example 7.

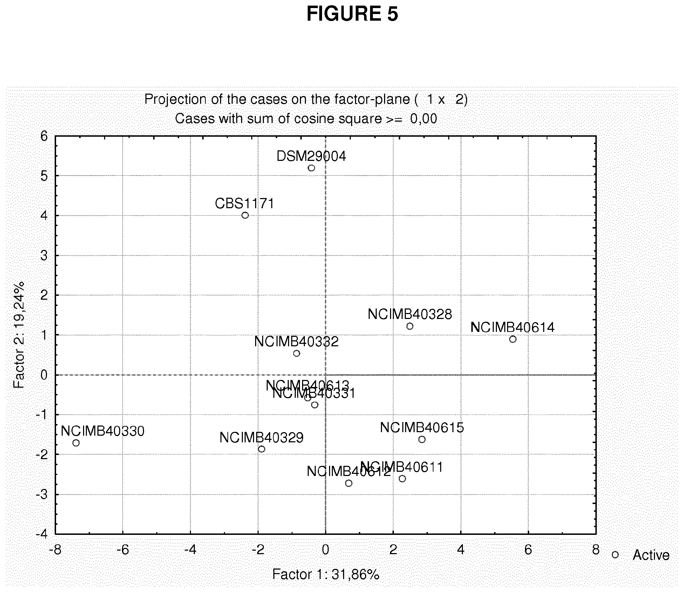

**Figure 5** shows the graphical representation of the Principal Component Analysis, as described in Example 8.

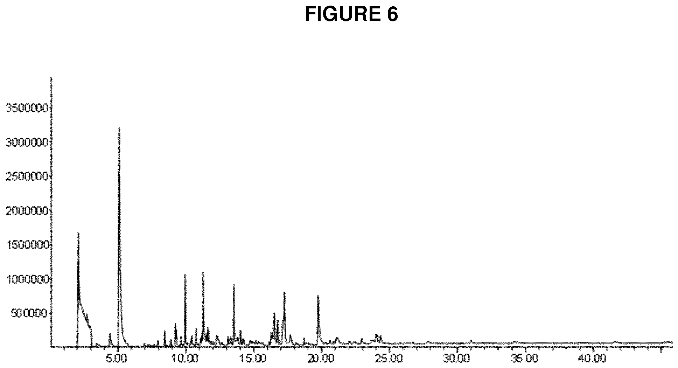

**Figure 6** shows, in a reproduction, one of the two replicated chromatographic profiles representing the volatile compounds obtained by fermenting the M4 medium based on barley and wheat malt extracts with the strain *Saccharomyces cerevisiae* DSM29004 for 72 hours at 27°C in an agitated flask, as reported in Example 10.

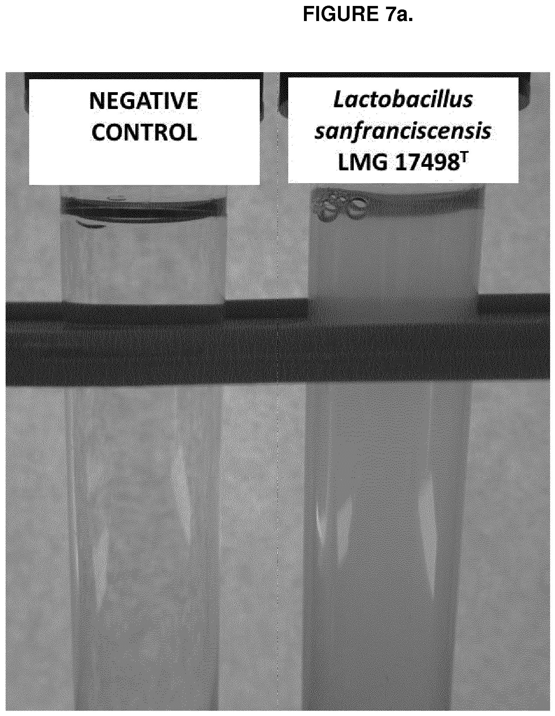

**Figure 7a****.** shows a liquid culture of the strain *Lactobacillus sanfranciscensis* LMG 17498^{T} in the medium DSM 225 (known also as "Dough Like Medium") produced according the protocol released by DSMZ and based on a fresh yeast extract from the strain DSM 29004 as described in Example 14.

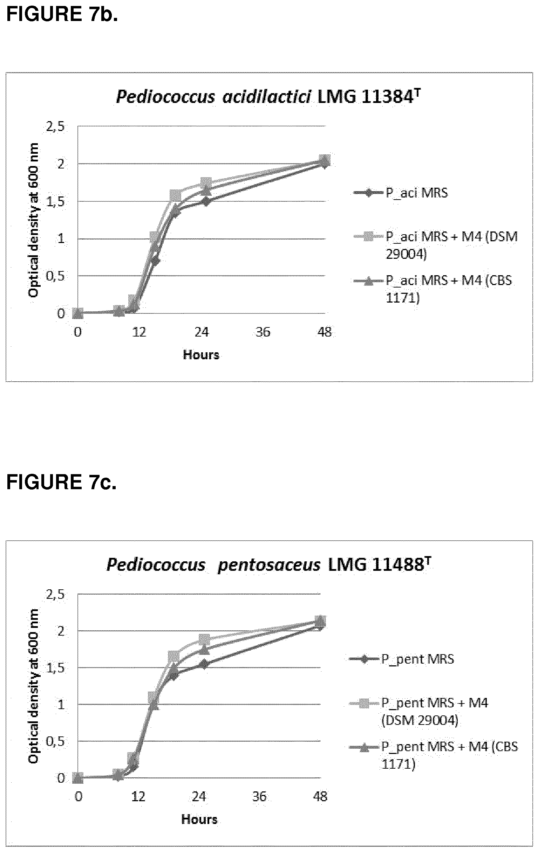

**Figure 7b****.** shows the kinetics of growth of *Pediococcus acidilactici* LMG 11384^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.
**Figure 7c****.** shows the kinetics of growth of *Pediococcus pentosaceus* LMG 11488^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.

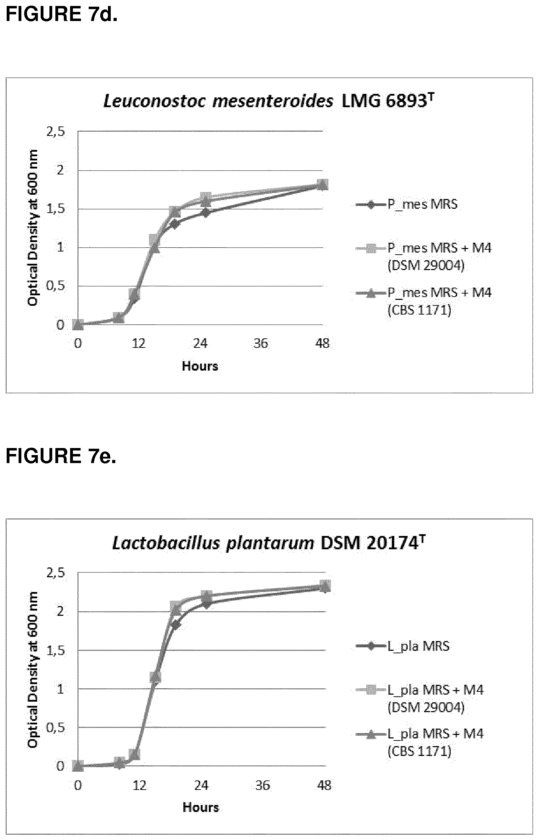

**Figure 7d****.** shows the kinetics of growth of *Leuconostoc mesenteroides* LMG 6893^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.
**Figure 7e****.** shows the kinetics of growth of *Lactobacillus plantarum* DSM 20174^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.

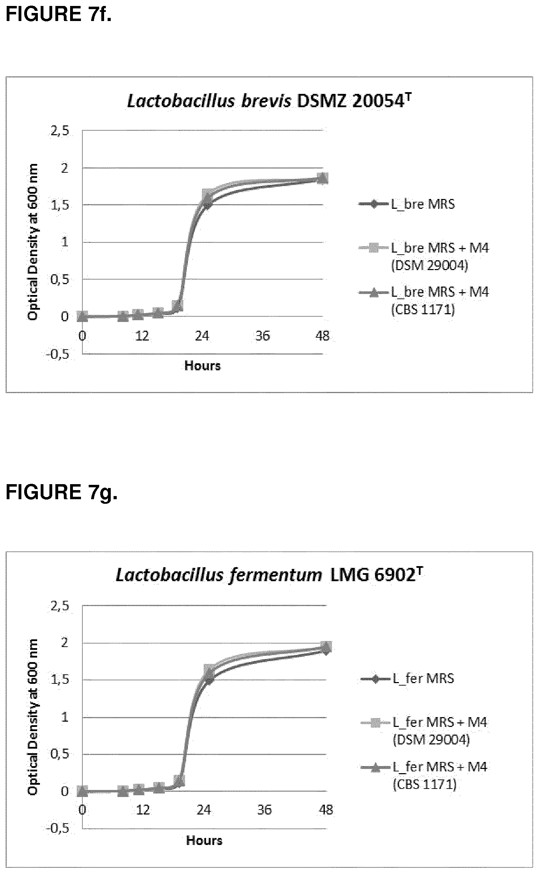

**Figure 7f****.** shows the kinetics of growth of *Lactobacillus brevis* DSM 20054^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.
**Figure 7g****.** shows the kinetics of growth of *Lactobacillus fermentum* LMG 6902^{T} in MRS medium supplemented, at the ratio 1:10 v/v, with the discarded medium obtained after the growth of the yeast strains DSM 29004 and CBS 1171^{T} in M4 medium. MRS without supplements is the control medium, as described in Example 14.

### Detailed description of the invention

The invention therefore relates to a yeast strain of the species *Saccharomyces cerevisiae,* filed on 01.07.2014 with accession number DSM 29004 at the International Deposit Authority Leibniz-Institut DSMZ - Deutsche Sammlung von Mikroorganismen und Zellkulturen GmbH.

As non-limiting example, a distinctive hallmark of the strain *Saccharomyces cerevisiae* DSM 29004 and of the essentially derived strains is provided by microsatellite DNA profile, defined by the analysis of length polymorphisms at *loci* characterized by the presence of *Simple Sequence Repeats* or SSR.

The strain according to the present invention is genotypically characterized and is identifiable in a clear and defined manner by specific traits identified within the genome. This strain has evolved spontaneously, without any direct intervention or genetic manipulation, and has the relevant characteristics for industrial application typical of a "Low temperature inactivation" or *LTi* strain, represented by the ability of being stored in an inactive but viable form at low temperatures (in a range between 0°C and 8°C) and reactivated simply by increasing the environmental temperature to a range between 12°C and 38 °C, preferably between 25 °C and 28°C. Reactivation is manifested as consumption of the available sugars and production of carbon dioxide which, under suitable conditions, makes possible to carry out leavening with the desired alveolation of the fermented dough.

*Saccharomyces cerevisiae* DSM 29004 strain from a spontaneously leavened dough (sourdough) of Italian origin, isolated by serial dilutions in saline followed by culture in selective *Saccharomyces* medium and refreshed periodically by addition of a mixture of water and unleavened flour, was filed in the Leibniz-Institut DSMZ - Deutsche Sammlung von Mikroorganismen und Zellkulturen GmbH on July 1, 2014 and was assigned the deposit number DSM 29004.

The *Saccharomyces cerevisiae* yeast strain of according to the present invention remains therefore surprisingly inactive, but vital for a long time, at temperatures below 8°C and then resumes normal metabolic activity, with production of carbon dioxide that allows the leavening process, when it is returned to temperatures optimal for growth, and in this its behaviour is dramatically distinct from "known" brewer's yeasts characterized by the aforementioned behaviour and drawbacks with respect to storage of raw doughs.

In order to verify and ascertain the characteristics of the DSM 29004 strain, and to rule out the overlap of this strain with the strains described in the prior art, a genotype comparison was performed between DSM 29004 and the *S. cerevisiae* strains NCIMB 40611, NCIMB 40612, NCIMB 40613, 40614, NCIMB 40615, NCIMB 40328, NCIMB 40329, NCIMB 40330, NCIMB 40331, NCIMB 40332 and CBS1171 ^{T}. Example 8 describes the experimental steps and describes the results shown in Figures 2b .

In Figure 2b it is clearly shown that the strain *Saccharomyces cerevisiae* DSM29004 in lane 11 is different from the other strains reported in the prior art because of three specific bands (band A, band B and band C) in the microsatellite DNA pattern, comprised in the range between 350 bp and 500 bp.

The results clearly indicate that DSM 29004 is a novel *Saccharomyces cerevisiae* yeast strain with improved characteristics.

In a preferred form of realization, the yeast strain of the species *Saccharomyces cerevisiae,* deposited with accession number DSM 29004 at the DSMZ on 01.07.2014 and the essentially derived strains, are characterized in that said strain remains viable for at least 20 days at temperature comprised between 0°C and +8°C, and said strain subsequently shifted to a temperature between +12°C and +38°C shows fermentative vigour and growth activity. The substantial absence of carbon dioxide production is evident in the case where the yeast is added to a dough made of flour and water. Said mixture maintained between 0°C and 8°C does not result in the swelling of a sealed package, a clear sign that gas production by yeast tends to zero On the other hand, the fermentative vigour becomes evident when the same dough, after 20 days of storage in a sealed package at variable temperatures in a range comprised between 0°C and 8°C, is left at room temperature (20-25°C) for about 20 minutes reaching a volume that is 3-fold its original volume, thus showing an excellent leavening action of the yeast. Recovery of the yeast's fermenting activity at 20-25°C temperatures (room temperature), after a step of refrigerated storage, makes possible to have finished products characterized by good alveolation and softness and crispness of the dough, at the same time. *Saccharomyces cerevisiae* DSM 29004 strain prove to be extremely advantageous for industrial application in fermenting activities, when used as a food ingredient.

In a further form of realization the yeast strain of the species *Saccharomyces cerevisiae,* deposited with accession number DSM 29004 at the DSMZ on 01.07.2014, is characterized in that said strain remains viable for at least 30 days at a temperature between 0°C and +6°C, and subsequently shows fermentative vigour and growth activity at temperature comprised between 12°C and 38°C, preferably between 25°C and 28°C.

The substantial absence of carbon dioxide production is evident in the case where the yeast is added to a dough made of flour and water. Said mixture maintained between 0° and 6°C does not result in swelling of a sealed package, a clear sign that gas production by yeast tends to zero. On the other hand, the fermentative vigour becomes evident when the same dough, after 30 days of storage in a sealed package at variable temperatures in the range comprised between 0°C and 8°C, is left at room temperature (20-25°C) for about 20 minutes reaching a volume that is 2.5-fold its original volume, thus demonstrating a significant leavening action of the yeast.

This feature is also called "*Low Temperature inactivation*"*,* abbreviated as LTi. The LTi character is multi-factorial and depends on multiple loci defined "*Quantitative Traits Laci*" (QTL); the genotype associated with the phenotype of interest has not yet been described. Methods for targeted genetic manipulation cannot produce the desired result in these cases, thus better results are often provided by a "blind" manipulation strategy such as conjugation of selected strains by sexual reproduction. According to current knowledge, all the strains of the species *Saccharomyces cerevisiae* described to possess this feature have been obtained by genetic manipulation, particularly induced random mutagenesis followed by conjugation or production of hybrids with other selected strains. In one particular case, the process involved the fusion of strains obtained by mutagenesis with two yeast strains selected for high fermentative vigour and normally used in the field of bakery products. Naturally occurring strains with LTi characteristics isolated from naturally leavened dough of Italian origin were not previously known.

In a preferred aspect, the yeast strain according to the present invention is characterized in that said strain shows a greater fermentative vigour in dough leavening, when previously grown in a barley malt and wheat malt extract medium than when grown in a molasses-based medium, at a temperature comprised between +12°C and +38°C, preferably between +25°C to +28°C.

Preferably, said barley malt and wheat malt extract medium is in a concentration ranging from 0.1 g/l to 100 g/l preferably comprised from 40 g/l to 80 g/l and said molasses-based medium has a glucose concentration ranging from 0.1 g/l to 200 g/l preferably from 20 g/l to 100 g/l.

In a further embodiment, the invention relates to a solid or fluid dough comprising at least water, flour and yeast of the species *Saccharomyces cerevisiae* deposited with accession number DSM 29004.

Further advantageous characteristics of the strain according to the invention have been outlined.

Surprisingly the DSM29004 strain produced with the traditional Molasses-based medium releases fruity aromas. Molasses-based medium generally has an unpleasant odour and so has the yeast grown on that medium. This usually causes a problem with the flavour of the dough which contains yeast grown on molasses-based media. Surprisingly the odour of the strain according to the invention, grown in molasses-based medium for at least 72 hours is substantially more pleasant than normal strains of *Saccharomyces cerevisiae* and this adds pleasant flavours to the dough, instead of worsening the flavour of the same dough (as reported in Example 9).

After the growth of the DSM 29004 strain in a barley malt extract and wheat malt extract supplemented with nitrogen sources, other salts and vitamins (as shown in Table 8), the resulting discarded medium was analysed for the content of alpha-aminic nitrogen (the nitrogen source also known when referred to yeast as FAN or free amino nitrogen, and as alpha-aminic nitrogen for bacteria) and it was found that the medium discarded from the growth of DSM 29004, was characterized by a higher content of alpha-aminic nitrogen compared to the other tested strains as described in Example 13. Alpha-aminic nitrogen is a source of essential factors for the growth of microorganisms, such as lactic acid bacteria, especially the lactic acid bacteria that are well adapted to dough and sourdough.

The presence of lactic acid bacteria is essential to improve dough texture and flavours, giving it a more natural fragrance (as describe in Example 14).

It was observed that the medium shown in Table 8, once discarded after the growth of DSM29004 surprisingly has the capacity of enhancing the growth of lactic acid bacteria belonging to the *Pediococcus* genus, at the same time without having negative effects on the growth of the normal growth of the other lactic acid bacteria. It was also observed that said discarded medium is characterized by the absence of sulphur dioxide, which however is usually produced by yeast during fermentation. The presence of sulphur dioxide inhibits the growth of lactic acid bacteria, thus is not advantageous in dough fermentation (Example 12) and would represent an insurmountable obstacle in the case the discarded medium was added to the dough together with the yeast strain as a supplement. On the contrary, the medium discarded after the growth of DSM29004, being substantially free of sulphur dioxide, is suitable for being added to a dough as a supplement, thus exploiting its aforementioned capacity of enhancing the growth of lactic acid bacteria.

At the same time, the presence of barley and wheat malt extracts in the medium (as shown in Table 8) induces the expression of the genes related with maltose fermentation. Maltose is the sugar mainly present in baked products dough. Thus the strain DSM 29004 grown on a medium such as the one shown in Table 8, once in a baked product dough, will use the maltose more efficiently and will display more promptness, more quickness and more vigour, than the one grown on molasses-based medium, thus favouring the dough leavening, (see Example 11). This characteristic of better leavening of the strain DSM 29004 grown on a medium such as the one in Table 8 represents a consistent advantage in the use of Lti strains, since it is known that these strains show, even at temperatures that are usually optimal for *Saccharomyces cerevisiae* fermentation, a lower metabolic activity, and this usually carries to insufficient leavening or long time needed for decent leavening. Baked products such as bread, pizza, flat bread (focaccia), savoury scones (tigelle) but also leavened products suitable for frying products such as fried puffy dough rectangles and fried dumplings are obtained from flour based doughs. Embodiments involve production of a dough that can be in solid or fluid form. The main difference between these doughs is the greater amount of water that is added as ingredient of the liquid mixture.

In particular, fluid dough refers to a mixture with viscosity, as measured by a Brookfield rotational viscometer, comprised between 47,000 cP (mPa·s) and 800 cP (mPa·s) at 50 rpm, with R7 and R3 impeller, respectively, or between 36,000 cP (mPa·s) and 600 cP (mPa·s) at 100 rpm, with R7 and R3 impeller respectively, in each case at 20°C temperature. This condition allows to obtain a sliding fluid mass, that in particular becomes distributed by gravity in the available space once it is poured onto the respective support means (baking pan).

In particular it is provided that such doughs contain flour, primarily wheat flour, especially a hard or soft wheat flour or a blend thereof. Moreover, the wheat flour is preferably of "0"type. It must however be understood that flour from other sources may be also employed, preferably spelt flour and possibly other flours such as rice, buckwheat, rye or other gluten-free flours. Flours from organic farming could also be used.

It is provided that the solid dough contains flour in a proportion comprised between 50% and 70% by weight of dough, and it is however preferable a proportion comprised between 54% and 67% by weight of dough. It is provided that the solid dough contains said flour in a proportion comprised between 43.5% and 60.6% by weight of dough and preferably between 44.5% and 50.5% by weight of said dough. In particular, in order to obtain a fluid dough with the aforementioned characteristics, said flour is such to display a force equal to or greater than 220W as measured by the Chopin alveograph.

It is intended that these doughs contain water. For solid dough, water is added to flour in a proportion comprised between 30% and 40% by weight of dough and preferably in a proportion comprised between 33% and 36% by weight of dough. For fluid dough, water is added in a proportion comprised between 35.5% and 56.5% by weight of dough, however preferably in a proportion between 44.5% and 50.9% by weight of dough.

To obtain a solid dough, the yeast DSM 29004 or an essentially derived strain is added in a proportion comprised between 0.4% and 2.4% by weight of dough, preferably in a proportion comprised between 0.8 % and 1.3% by weight of dough. To obtain a fluid dough, the yeast strain DSM 29004 or an essentially derived strain is added in a proportion up to a maximum of 1.8% by weight of dough, preferably in a proportion comprised between 0.6% and 0.8% by weight of dough.

Between 1·10⁵ CFU/g of dough and 1·10⁷ CFU/g of dough, preferably 5·10⁶ CFU/g of dough, of the yeast DSM 29004 or an essentially derived strain are inoculated. Furthermore, salt or sodium chloride is added in order to obtain said dough. In particular, said salt is in an amount up to a maximum of 2% by weight of dough, preferably in an amount comprised between 0.7% and 1.8% by weight of dough. Moreover, to obtain said dough, oil and/or dietary fat, or a mixture of oils and/or dietary fats, are preferably added. Preferably olive oil and more preferably extra virgin olive oil is used. Said oil and/or dietary fat is added in a proportion up to a maximum of 6% by weight of dough, preferably in a proportion comprised between 1.9% and 5.5% by weight of dough. Oils from organic farming could also be used. In addition, malt extract or malted grain flour is preferably added to obtain said dough. Said malt extract or malted grain flour is a proportion up to a maximum of 0.8% by weight of dough. Malt extract or malted grain flour from organic farming could also be used.

Preferably, said dough also contains a leavening aid. In particular, said leavening aid comprises a dried base of acid sourdough of flour, in particular made of soft wheat, gluten, L-ascorbic acid and alpha-amylase. In particular, said leavening aid is in an amount lower than or equal to 0.6% by weight of dough. A "BIO" processing aid could also be used.

Moreover, said mixture preferably contains deactivated yeast. In particular, said deactivated yeast is in an amount lower than 2% by weight of the dough.

A first procedure for making a bakery product, in particular pizza, involves mixing the flour together with said aid and said malt, and the addition of said yeast and said deactivated yeast.

Water and oil or fat are added in a subsequent step and a first mixing of the dough is carried out. Salt is then added and a second mixing is carried out, thus achieving the stage of a dough for baking which is ready for further processing and is in solid, fluid or substantially fluid form according to the recipe followed for its production.

In the case of fluid dough, this is poured into a baking pan and freely spreads in the containment space defined by the pan. In the case of a solid dough, this is mechanically rolled out always within the respective pan. Said rolled out dough can be defined as the base for bakery product.

The so obtained base is delivered to a leavening cell, where it is subjected to convenient leavening. In particular, said leavening is done in an environment suitably controlled for temperature and humidity, and in particular is extended for a time interval of about two hours.

Subsequently, fluid or substantially fluid tomato, particularly in the form of tomato sauce, pulp or paste, is spread on the upper surface of said base.

In a next step the base topped with tomato is cooked in a special oven.

Then a topping is added onto the upper surface of the base, preferably consisting even simply of pieces of mozzarella cheese.

Obviously this step would not be present in the preparation of products such as flat bread, bases for pizza or the like.

Subsequently the product could be packaged in the respective package.

Another very similar procedure may provide for a preliminary cooking step of the white base and the subsequent topping of the upper surface with tomato, mozzarella cheese and other desired toppings.

However, it is to be understood that also a corresponding flat bread (focaccia) could be obtained by using a process that provides for said steps carrying to completion the product base, obtained after leavening the product, followed for example by topping the upper surface with oil and other possible ingredients, and the corresponding cooking.

A second procedure to obtain a bakery product involves direct packaging of the dough, both in solid or fluid form, in a suitable package.

In this case, immediately after mixing the ingredients, the dough is divided into aliquots and pre-fermented at roughly 20-25°C temperature for a period of time from 30 to 60 minutes, preferably 30 minutes. The different aliquots of the dough are then cooled in a suitable cooling cell, until the core temperature of the product reaches below 4°C.

In a further aspect, the present invention comprises a dough divided into aliquots. Said aliquots can be pre-fermented at a temperature of about 20-25°C for a period of time from 30 to 60 minutes, preferably 30 minutes.

Subsequently, individual aliquots are packaged into special heat-sealed or not-sealed packages. Packaging can take place under conditions of modified atmosphere or ordinary atmosphere. As for the packaging in a modified or protective atmosphere, the gas mixture includes carbon dioxide used in proportions from 0% to 60% and nitrogen used in proportions from 0% to 80%, preferably 50% carbon dioxide and 50% nitrogen.

In this second procedure, the final consumer stores the packaged dough directly in the refrigerator or in any case at temperatures in a range of from 0°C to 8°C, preferably at temperatures in a range of from 0°C to +6°C.

The product is stored under the above conditions for a period of 20 days at temperatures in a range from 0°C to +8°C, preferably for a period of 30 days at temperatures in a range of from 0°C to +6°C.

Within this storage period, the packaged dough is used for production of a bakery product, such as bread, pizza and flat bread (focaccia), but also for production of leavened products suitable for frying.

The solid dough is removed from the package and allowed to stand at room temperature (20-25°C), preferably for about 20-30 minutes. After this time the dough is rolled on a suitable support sprinkled with flour and brought to the desired size while maintaining a sheet thickness of about 4-5 mm. Thickness and size are variable, however, depending on the final product to be obtained

For the production of bakery products, the dough is then garnished on the upper surface with different ingredients depending on the product to be obtained. In case of production of a pizza, the rolled out dough will be topped with tomato in liquid or substantially liquid form, in particular in the form of tomato sauce, pulp or a paste, and with pieces of mozzarella cheese and other desired toppings. It is also possible to plan a step of baking in the oven the rolled out dough alone, followed by filling of the upper surface.

In case of production of a focaccia or flat bread, the upper surface of the base will be oiled with olive oil and sprinkled with salt as desired.

The topped rolled out dough, in both cases, is subjected to cooking. The bakery product is thus completed.

For production of leavened products suitable for frying, the phase for preparation of the dough corresponds to that described for baked products. The difference is in cooking the product, which is not done in the oven but in frying oil and/or fat.

Use of a fluid dough involves the opening of the package and the dough must be poured into an oiled baking pan. Dough viscosity allows its distribution by gravity in the available space inside the support. In case, it is possible to facilitate spreading by a slight mechanical action in order to cover the entire surface of the pan.

The dough is allowed to rest at room temperature (20-25°C) in the baking pan covered with a plastic film to prevent surface hardening.

At this point it is possible to proceed with the production of baked products with the same preparation modalities described above.

A further aspect of the dough according to the present invention is a dough wherein said yeast strain DSM 29004 of the species *Saccharomyces cerevisiae* is in the form of viable cells.

Said strain and the essentially derived strains are in the form of viable cells. The strain in question can be used to obtain said dough as mixture with other yeasts and viable, non-viable microorganisms and extracts or lysates of yeast or other microorganisms.

According to a further aspect of the present invention, the dough is a mixture wherein said yeast of the species *Saccharomyces cerevisiae* is mixed with other yeasts and/or viable, non-viable microorganisms, extracts or lysates from other yeasts and other microorganisms.

According to yet a further aspect, the dough of the present invention is characterized in that it does not show package swelling, development of abnormal odour and taste or formation of liquid inside the package when it is stored at a temperature between 0°C and +8°C for 20 days, or preferably at a temperature between 0°C and +6°C for 30 days.

According to yet another aspect, the invention relates to the use of the yeast strain of the species *Saccharomyces cerevisiae,* deposited with accession number DSM 29004 at the International Deposit Authority Leibniz-Institut DSMZ - Deutsche Sammlung von Mikroorganismen und Zellkulturen GmbH, for the production of solid or fluid doughs.

Uses comprise the application for solid or fluid dough of the strain *Saccharomyces cerevisiae* DSM 29004 and the essentially derived strains. As a non-exclusive example, an industrial use of *Saccharomyces cerevisiae* strain DSM 29004 or the essentially derived strains is represented by mixing with flour-based dough and storage in a viable but inactive state at temperatures comprised between 0°C and 8°C, with the property to be reactivated to produce carbon dioxide when it is returned to a temperature range optimal for growth. *Saccharomyces cerevisiae* strain DSM 29004 and the essentially derived strains are characterized by the presence in their genome of nucleotide sequences that are unique in terms of sequence of bases, size, copy number or location with respect to the flanking regions, which are detectable and reproducible by molecular biology techniques.

According to yet another aspect, the invention relates to a process for preparation of a refrigerated dough in solid or fluid form, wherein the preparation consists in the mixing at least water, flour and yeast of the species *Saccharomyces cerevisiae* deposited with the accession number DSM 29004 at the International Deposit Authority Leibniz-Institut DSMZ - Deutsche Sammlung von Mikroorganismen und Zellkulturen GmbH, wherein said dough is packaged in a container. Advantageously, in a preferred form of realization of the process according to the present invention, said dough is packaged in a container with a modified atmosphere of nitrogen (N₂) from 0 to 80% and of carbon dioxide (CO₂) from 0 to 60%, or in a sealed container containing ordinary atmosphere.

The doughs obtained according to the present invention can be packaged in heat sealed or non-heat-sealed trays, and even at the expiration date (i.e. after 30 days) no package swelling, development of abnormal odours and taste, formation of liquid inside the package are observed. Once the package is opened, the product is left to rest and leaven at room temperature for about 20 minutes before rolling out the dough. This timing allows the yeast present in the dough to regain its fermentative vigour and makes possible to achieve a finished product characterized by good alveolation, pleasantness and softness of the dough.

In a still further embodiment the invention relates to a process for the preparation of a medium having a high alpha-amino nitrogen concentration (also referred to herein as "discarded medium"), comprising the steps of:
a. growing the yeast strain DSM 29004 in a barley malt and wheat malt extract medium for at least 36 hours;
b. separating the yeast from the medium, preferably by filtration or by centrifugation;
c. obtaining a medium having a high alpha-amino nitrogen concentration higher than 300 mg/l, wherein the alpha-amino nitrogen is not added to the medium but results from the yeast growth.

A characteristic of the medium is that DSM 29004, grown in the medium described in the paragraph above, releases during fermentation, preferably: octanoic acid ethyl ester, phenylethyl alcohol, acetic acid phenylethyl ester, benzeneacetaldehyde, 4-methyl-2-heptanone, octanal, nonanoic acid ethyl ester.

In the examples is described the use of the medium as a supplement for dough and as a supplement for enhancing dough fermentation of lactic acid bacteria species, preferably chosen from the group consisting of *Lactobacillus brevis, Lactobacillus fermentum, Lactobacillus plantarum, Lactobacillus sanfranciscensis, Leuconostoc mesenteroides Pediococcus* acidilactici and *Pediococcus pentosaceus.* Preferably the lactic acid bacteria belong to the *Pediococcus* spp genus.

Examples of embodiments and preferred procedures of the present invention are described below to illustrate the invention.

### EXAMPLES

### Example 1: Methods to analyze biodiversity: microsatellite DNA

*Saccharomyces cerevisiae* strain DSM 29004 belongs to the species *Saccharomyces cerevisiae* as evidenced by the positive result (Figure 1) of species-specific PCR (Torriani et al., 2004). Moreover, to the best knowledge of the prior art, it is characterized by a unique genotype described, in the preferred forms, by:
- profile analysis of microsatellite DNA by 3% weight/volume agarose gel electrophoresis (Figure 2) of amplification products of *multiplex PCR* amplifying SC8132X, YOR267C, SCPTSY7 (Vaudano & Garcia-Moruno, 2008) and C5 (Legras et al., 2005) *loci.* The primers used were: SC8132X FW and SC8132X RV each at 0.75 µM concentration (Vaudano & Garcia-Moruno, 2008); YOR267C FW and YOR267C RV each at 0.5 µM concentration (Vaudano & Garcia-Moruno, 2008); SCPTSY7 FW and SCPTSY7 RV at 2.0 µM concentration (Vaudano & Garcia-Moruno, 2008) and finally the primers C5 FW and C5 RV at 0.5 µM concentration (Legras et al., 2005). The reaction was performed in 20µl volume using 1.5U of DNA polymerase GoTaq (Promega) in 1x buffer with addition of magnesium chloride (MgCl₂) at 3.1 mM concentration and tri-phosphate nucleotides (dNTPs) each at 0.4 mM concentration. Amplification was in the Nexus Mastercycler Gradient instrument (Eppendorf) with a thermal program consisting of an initial denaturation step at 94°C for 4 minutes followed by one cycle at 94°C for 30 seconds, 56°C for 45 seconds and 72°C for 30 seconds, repeated 34 times, followed by a final extension step at 72°C for 10 minutes.
- microsatellite DNA profile analysis for YGL139W, YFR028C, YGL014W, YOL109W, YML091C, YLL049W, YBR240C, YDR160W, YPL009C, YOR267C *loci,* obtained by amplification in separate PCR reactions, using primers described in the literature (Richards et al. 2009), followed by separation of the fragments in a capillary (Figure 3). The profile analysis in the capillary was performed with the Peak Scanner 2.0 software with the following parameters: "size standard: GS500 (-250)" and "analysis method: default sizing - NPP".

The microsatellite DNA *loci* are few hundred bases sequences scattered in the genome, consisting of tandem repeats of short 1-6 bases motifs. They are also known as *Simple Sequence Repeats* or *SSR.* These *loci* are widely used as genetic markers due to their diffusion, high degree of polymorphism and high reproducibility and reliability of their detection assays (Dutech et al., 2007). These are common *loci* among eukaryotes (yeasts, molds, plants and higher animals) and are rarely present in bacterial species. Polymorphism occurs essentially at two levels: presence / absence of single *loci* and length variations in each locus due to the number of repetitions of a single tandem repeat motif.

Microsatellite DNA analysis consists in PCR amplification of the *loci* of interest. The primers used for PCR are designed in conserved regions flanking the *locus* to be analyzed. If the *locus* is present, a portion of DNA containing the tandem repeat motifs is obtained as PCR product. PCR product length varies depending on the allelic form that is present in the genome analyzed. The presence of several *loci* and their size is displayed by agarose gel electrophoresis. If the primers used are labelled with a fluorescent molecule, the run can be performed in the capillaries used for *Sanger* sequencing. In both agarose gel and capillary analysis, the migration of the PCR products shows the size of the bands according to their relative mobility. Dozens of different loci are known in *Saccharomyces cerevisiae* (Legras et al., 2005). Some have a limited number of allelic forms (variants of the same locus observed in strains of the same species) and are poorly discriminating, while others are characterized by a greater number of allelic forms and are therefore more discriminating (Legras et al., 2005; Vaudano & Garcia-Moruno, 2008).

To the best of prior art knowledge, the most comprehensive database of microsatellite DNA profiles of strains belonging to the species *Saccharomyces cerevisiae* is available for consultation and consists of 246 profiles generated by capillary analysis of 11 loci identified based on their higher allelic frequency, with the exception of the locus *mat a* / mat α that only has two forms. The database generated by Richards and colleagues (2009), contains microsatellite DNA profiles from strains isolated in the field and commercial strains from the wine industry and other sectors (Richards et al., 2009). In parallel projects other similar protocols have been developed that confirmed the high capacity of microsatellite DNA loci to differentiate different strains of the species *Saccharomyces cerevisiae,* however, are no database is available for consultation (Legras et al., 2007). The DSM 29004 strain has a unique microsatellite DNA profile, compared to all other strains described in the database produced by Richards et al., 2009.

### Example 2: Comparison of the behaviour of the DSM 29004 strain with the type strain of Saccharomyces cerevisiae

In order to differentiate the properties of DSM 29004 strain from those of standard *Saccharomyces cerevisiae* strains, *Saccharomyces cerevisiae* DSM 29004 strain and *Saccharomyces cerevisiae* CBS 1171^{T} type strain were used for the preparation of two identical pizza doughs that were different only for the yeast strain inoculated, and consisted of flour, water (52% by weight of the flour), extra virgin olive oil (8.42% by weight of the flour), salt (1% by weight of the flour). The two yeast strains were added to the kneading water at a concentration of about 1 10⁶ CFU/g. The two doughs thus obtained were packed in sealed packages in a protective atmosphere (50% nitrogen and 50% CO₂), stored at 6° C and monitored over time.

### Behaviour of DSM 29004 strain

With regard to the dough with DSM 29004 strain, during the first 15 days of refrigerated storage a decrease of about 1 log cycle was observed in terms of CFU/g, from 1 10⁶ CFU/g to 1 10⁵ CFU/g while during the remaining period, up to 30 days of storage, a slowdown in the decline was observed with a change from 1 10⁵ CFU/g to 5 10⁴ CFU/g. During these 30 days the sealed package never showed any sign of swelling or increased internal pressure.

Yeast viability and ability to give rise to well structured and alveolated doughs was also checked 15 days and 30 days after the preparation.

In both tests, the dough was extracted from the package, rolled out in the baking pan and left at room temperature (23°C) for 30 minutes.

In the test carried out at day 15, the height of the raw dough increased 3-fold its original height, demonstrating an excellent activity of the yeast. The dough leavened for 30 minutes increased further, albeit slightly, during cooking. The baked product showed a large and structured alveolation that was quite similar to that obtainable with a dough prepared the same day.

In a test carried out at 30 days, the height of the raw dough increased to 2.5- fold its original height, showing significant activity of the yeast. The dough leavened for 30 minutes increased further, albeit slightly, during cooking. The cooked product still showed a large and structured alveolation that was comparable to that obtained in the test carried out at day 15.

In no case the product had strange odours of its ingredients or unusual colors.

### Behaviour of the type strain of Saccharomyces cerevisiae

On the contrary, *Saccharomyces cerevisiae* strain CBS 1171^{T} caused unacceptable swelling of the sealed package (and breakage in many cases) even after 3 days of storage at +6°C, thus showing a strong activity of the yeast at the temperature of 6°C. After opening the package, the dough had abnormal smell and taste, characterized by acid and alcoholic odor. The dough structure was weakened to the point of making impossible to proceed rolling it out.

After these three days, there was an increase of 2 log cycles, from 1.10⁶ CFU/g to 1.10⁸ CFU/g of dough.

Therefore there is a clear difference in behaviour exhibited by strain DSM 29004 and type strain, with regard to both the fermenting activity at 6°C and quality of leavening once the dough was extracted from the package.

### Example 3: preparation of a solid dough for pizza

*Saccharomyces cerevisiae* DSM 29004 strain is used for preparation of a solid dough for pizza according to the following recipe shown in Table 1:

**Table 1**

| INGREDIENTS: | INGREDIENTS% |
|---|---|
| FLOUR 60% | 60 % |
| WATER 45% | 33 % |
| EXTRA VIRGIN OLIVE OIL 2.9% | 3 % |
| SALT 1.5% | 1.8 % |
| YEAST 0.8% | 1 % |
| BARLEY MALT 0.3% | 0.3 % |
| PROCESSING AID 0.5% | 0.3 % |
| DEACTIVATED YEAST 0.6% | 0.6 % |

The yeast is inoculated at a concentration of 510⁶ CFU/g of dough.

This dough is portioned in balls weighing about 250 g.

Each ball dough is cooled to a temperature of about 0°C/4°C and is packaged in a thermoformed tray in modified atmosphere (50% N₂ and 50% CO₂). The package is then stored in a refrigerator at a temperature of about 6°C for 20 days. After twenty days from the production date, the dough ball is removed from the package and left at room temperature (20-25°C) for about 20 minutes. After said interval the dough is rolled out on a floured cutting board until reaches the size of a round base of about 28 cm). The latter is then transferred onto greaseproof paper and garnished with tomato paste or chopped tomatoes, chopped mozzarella cheese and other toppings to taste. The pizza is then cooked on the middle grill of the oven preheated to 230°C and set on ventilation mode for 6-8 minutes until the mozzarella cheese is completely melted.

This results in a pizza that has a soft and aveolated crumb and crispy edge and crust.

### Example 4: preparation of a solid dough for focaccia (flat bread)

*Saccharomyces cerevisiae* DSM 29004 strain is used for preparation of a solid dough according to the following recipe shown in Table 2:

**Table 2**

| INGREDIENTS: | INGREDIENTS% |
|---|---|
| FLOUR 60% | 60 % |
| WATER 45% | 33 % |
| EXTRA VIRGIN OLIVE OIL 2.9% | 3 % |
| SALT 1.5% | 1,8 % |
| YEAST 0.8% | 1 % |
| BARLEY MALT 0.3% | 0.3 % |
| PROCESSING AID 0.5% | 0.3 % |
| DEACTIVATED YEAST 0.6% | 0.6 % |

The yeast is inoculated at a concentration of 5 10⁶ CFU/kg of dough.

This dough is portioned into balls weighing about 400g.

Each dough ball is cooled to a temperature of about 0°C and is packaged in a thermoformed tray in modified atmosphere (50% N₂ and 50% CO₂). The package is then stored in a refrigerator at a temperature of about 6°C for 20 days.

After twenty days from the production date, the dough ball is removed from the package and left at room temperature (20-25°C) for about 20 minutes. After said interval the dough is rolled out on a floured cutting board until reaches the size of a baking pan (30x40 cm). The latter is then transferred on greaseproof paper, and is perforated with a fork to avoid excessive leavening during cooking. The surface of the base is then oiled with about 3 tablespoons of extra virgin olive oil and salt to taste. The flat bread is then cooked on the median grill of the oven, preheated to 200°C and set on ventilation mode, for about 14 minutes.

This results in a flat bread that looks well risen and characterized by a soft and alveolated crumb with crispy edge and crust.

### Example 5: preparation of a solid dough for fried puffy dough rectangles

*Saccharomyces cerevisiae* DSM 29004 strain is used for preparation of a solid dough for fried puffy dough rectangles according to the following recipe shown in Table 3:

**Table 3**

| INGREDIENTS: | INGREDIENTS% |
|---|---|
| FLOUR 60% | 60 % |
| WATER 45% | 33 % |
| EXTRA VIRGIN OLIVE OIL 2.9% | 3 % |
| SALT 1.5% | 1.8 % |
| YEAST 0.8% | 1 % |
| MALT BARLEY 0.3% | 0.3 % |
| PROCESSING AID 0.5% | 0.3 % |
| DEACTIVATED YEAST 0.6% | 0.6 % |

The yeast is inoculated at a concentration of 5x10⁶ CFU/g of dough.

This dough is portioned into balls weighing about 400g.

Each dough ball is cooled to a temperature of about 0°C and is packaged in a thermoformed tray in modified atmosphere (50% N₂ and 50% CO₂). The package is then stored in a refrigerator at a temperature of about 6°C for 20 days.

After twenty days from the production date the dough ball is removed from the packaging and left at room temperature (20-25°C) for about 20 minutes. After that time the dough is rolled out on a floured cutting board to obtain a rolled-out dough with a thickness of 2-3 mm The rolled-out dough is then cut into rectangles approximately 10 cm x 10 cm. Separately 500ml/1L of oil suitable for frying (olive oil or peanut oil) are preheated in a pan. Once the oil is hot, the rectangles of dough are immersed little by little for about 30 seconds. The puffy rectangles are then individually drained on paper towels.

A fried product is obtained that is characterized by strong dough leavening.

### Example 6: preparation of a fluid dough for the production of a pizza

*Saccharomyces cerevisiae* DSM 29004 strain is used for preparation of a fluid dough for the production of a pizza according to the following recipe shown in Table 4:

**Table 4**

| INGREDIENTS: | INGREDIENTS% |
|---|---|
| FLOUR TYPE 0 | 48.9 % |
| WATER 45% | 45 % |
| EXTRA VIRGIN OLIVE OIL 2.9% | 2.9 % |
| SALT 1.5% | 1.5 % |
| YEAST 0.8% | 0.8 % |
| PROCESSING AID 0.5% | 0.5 % |
| MALT EXTRACT 0.4% | 0.4 % |

The yeast is inoculated at a concentration of about 5x10⁶ CFU/g of dough.

Such fluid dough is divided into packages of 600 g and each package is sealed in modified atmosphere (50% N₂ and 50% CO₂). The package is stored in a refrigerator at a temperature between 0°C and 8°C, preferably 6°C for 20 days.

After twenty days from the production date the package is opened and 600 g of fluid dough are poured into a baking pan well-oiled with a tablespoon of olive oil. The dough is well distributed in all the available space of the pan and is left to stand at room temperature for 2 hours. The tray can be covered with a teflon film to avoid formation of surface films. After two hours the white base is cooked for about 16 minutes at 200°C in an oven set on ventilated mode. After cooking, the product is topped with 100g of tomato sauce and 100g of mozzarella cheese cut into pieces. At this point the product is cooked for 8-10 minutes at 200°C in an oven set on ventilated mode.

A pizza with a soft and alveolated crumb and crispy edge and crust is obtained.

### Example 7: panel test for verification of the overall pleasantness of the product

The baked products obtained following the above recipes and preparation methods are subjected to a "panel test" to confirm the fact that such products are characterized by a good alveolation, and at the same time softness and crispness of the dough.

The panel test or sensory analysis is a tool that allows, owing to the use of standardized techniques and procedures, the measurement and objective evaluation of all the characteristics of a food product perceived by the five human senses. Its systematic application, combined with chemical, physical and microbiological analyses, can identify a quality product satisfactory for consumers' taste preference.

A confirmation panel test was also performed (Figure 4) to confirm the overall pleasantness of the product from the organoleptic point of view. The products that were tested are white bases obtained from solid dough, pre-fermented for 30 minutes during the production phase, packaged in a protective atmosphere (50% N₂ and 50% CO₂) and stored for 21 days at +8°C The preparation of the finished product was made following the procedures indicated above.

The advantages achieved by the product of the present invention are evident from the detailed description and from the above examples. In particular, this product has proved to be surprisingly and advantageously suitable for the preparation of a solid or fluid dough that can be stored at temperatures below 8°C, even for more than 20 days, without qualitative changes of the dough and of the package, and makes possible, once it is shifted to room temperature, to obtain bakery products characterized by an optimal rise.

### Example 8: Genotype comparison of different Saccharomyces cerevisiae strains

Microsatellite DNA comparison of the following yeast strains was performed in order to verify if there is an overlap between the DSM 29004 strain according to the present invention and the prior art strains.

Strains tested: *Saccharomyces cerevisiae* DSM 29004, *S. cerevisiae* NCIMB 40611; *Saccharomyces cerevisiae* NCIMB 40612; *Saccharomyces cerevisiae* NCIMB 40613; *Saccharomyces cerevisiae* NCIMB 40614; *Saccharomyces cerevisiae* NCIMB 40615; *Saccharomyces cerevisiae* NCIMB 40328; *Saccharomyces cerevisiae* NCIMB 40329; *Saccharomyces cerevisiae* NCIMB 40330; *Saccharomyces cerevisiae* NCIMB 40331; *Saccharomyces cerevisiae* NCIMB 40332 and *Saccharomyces cerevisiae* CBS1171 ^{T}.

The strain *Saccharomyces cerevisiae* DSM 29004, isolated from a spontaneously fermented dough from Italy, is characterized by a unique genotype described, in the preferred forms, by:
profile analysis of microsatellite DNA by 3% weight/volume agarose gel electrophoresis (Figure 2) of amplification products of *multiplex PCR* amplifying SC8132X, YOR267C, SCPTSY7 (Vaudano & Garcia-Moruno, 2008) and C5 (Legras et al., 2005) *loci.* The primers used were: SC8132X FW and SC8132X RV each at 0.75 µM concentration (Vaudano & Garcia-Moruno, 2008); YOR267C FW and YOR267C RV each at 0.5 µM concentration (Vaudano & Garcia-Moruno, 2008);
SCPTSY7 FW and SCPTSY7 RV at 2.0 µM concentration (Vaudano & Garcia-Moruno, 2008) and finally the primers C5 FW and C5 RV at 0.5 µM concentration (Legras et al., 2005). The reaction was performed in 20µl volume using 1.5U of DNA polymerase GoTaq (Promega) in 1x buffer with addition of magnesium chloride (MgCl₂) at 3.1 mM concentration and tri-phosphate nucleotides (dNTPs) each at 0.4 mM concentration. Amplification was in the Nexus Mastercycler Gradient instrument (Eppendorf) with a thermal program consisting of an initial denaturation step at 94°C for 4 minutes followed by one cycle at 94°C for 30 seconds, 56°C for 45 seconds and 72°C for 30 seconds, repeated 34 times, followed by a final extension step at 72°C for 10 minutes.

The results clearly indicate that DSM 29004 is a novel *Saccharomyces cerevisiae* yeast strain with improved characteristics, which is characterized by a unique genotype as can be seen in the profile analysis of microsatellite DNA as shown in (Figure 2b).

More in detail, the DSM 29004 strain is different from the other strains reported in the prior art because of three specific bands (band A, band B and band C) in the DNA microsatellite pattern, comprised in the range between 350 bp and 500 bp.

### Example 9: comparison of the volatile compounds released by the Saccharomyces cerevisiae DSM 29004 strain, and the other strains, during the production in molasses-based medium by SPME GC-MS analysis

Strains tested: *Saccharomyces cerevisiae* DSM 29004, NCIMB 40611; NCIMB 40612; NCIMB 40613; NCIMB 40614; NCIMB 40615, NCIMB 40328; NCIMB 40329; NCIMB 40330; NCIMB 40331 ; NCIMB 40332 and CBS1171 ^{T}.

All the *S. cerevisiae* strains having LTi phenotype described in the prior art were thus compared to the *S.cerevisiae* strain DSM 29004 according to the present invention, by analyzing the aroma production, to verify the phenotype activity differences between the strains.

The strains were grown in YPD medium (Table 5). A culture of each strain in the mid-exponential phase was diluted to reach a concentration of 0.1 OD at the wavelength of 600 nm. The "Molasses-based" medium (Table 6) was independently inoculated with 100µl of such suspension of yeast cells at the concentration of 0.1 OD and incubated 72h at 27°C.

The following tables show the list of the components and their concentration in the media used to grow the yeasts strains in batch culture

**Table 5. Yeast Extract Peptone and Dextrose medium (YPD):**

| INGREDIENTS: | CONCENTRATION |
|---|---|
| YEAST EXTRACT | 10.0 g/l |
| PEPTONE | 20.0 g/l |
| DEXTROSE | 20.0 g/l |

**Table 6. Molasses-based medium for batch culture (MM);**

| INGREDIENTS: | CONCENTRATION |
|---|---|
| BEET MOLASSES | 100.0 g/l |
| (NH₄)H₂PO₄ | 4.35 g/l |
| (NH₄₎Cl | 2.02 g/l |
| K₂HPO₄ | 1.0 g/l |
| SODIUM CITRATE | 2.0 g/l |
| BIOTIN | 0.05 mg/l |
| CALCIUM PANTOTHENATE | 50.0 mg/l |
| INOSITOL | 250.0 mg/l |

At the end of said period, 3 ml of culture broth was transferred to sterile vials (10 ml capacity) for the volatile compound analysis. 10µl of a 1000 ppb stock of internal reference standard (4-methylpenthanol) were added to each vial before sealing it. SPME GC-MS analysis was performed on each sample. The experimental conditions are those described by Montanari et al. (2014).

The aromatic profiles obtained for each strain are reported in Table 7, in which each result is the average of three measurements. Each result is expressed as the ratio of area of the peak of and the area of the reference standard.

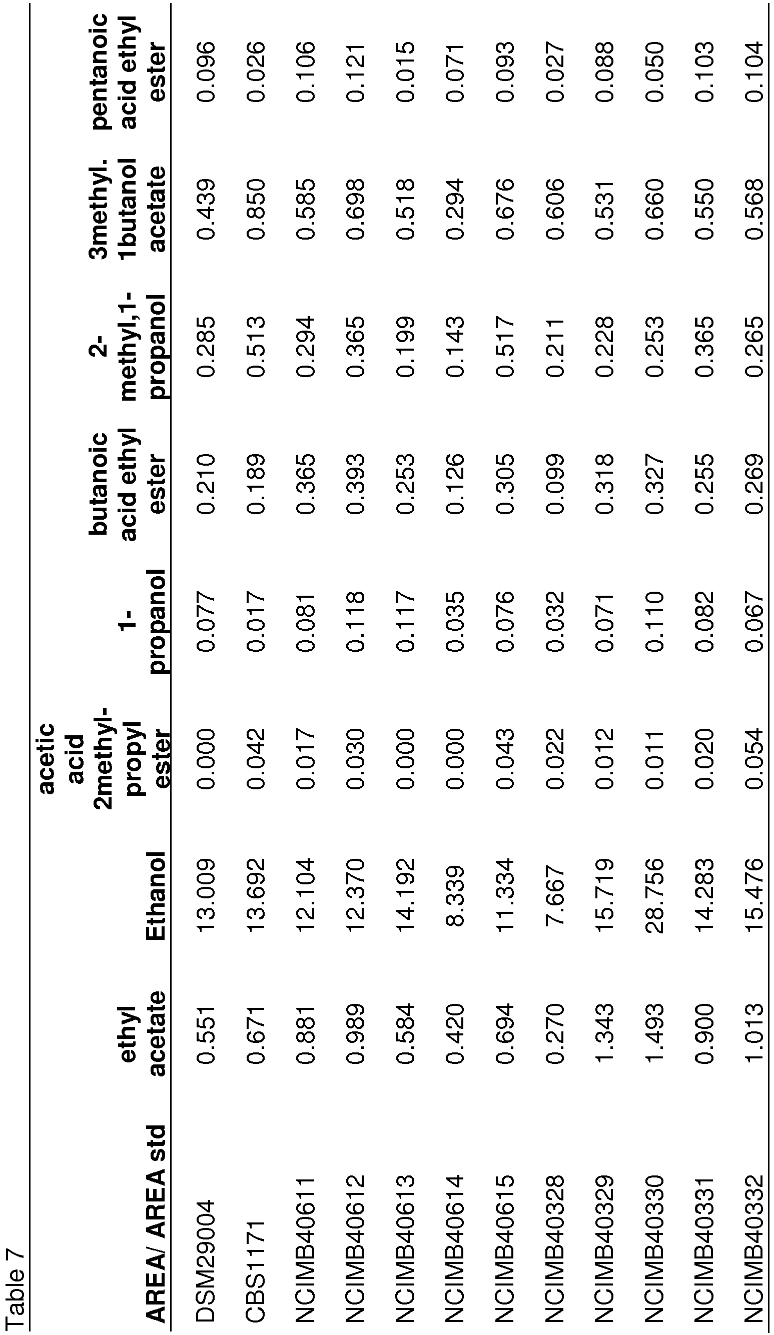

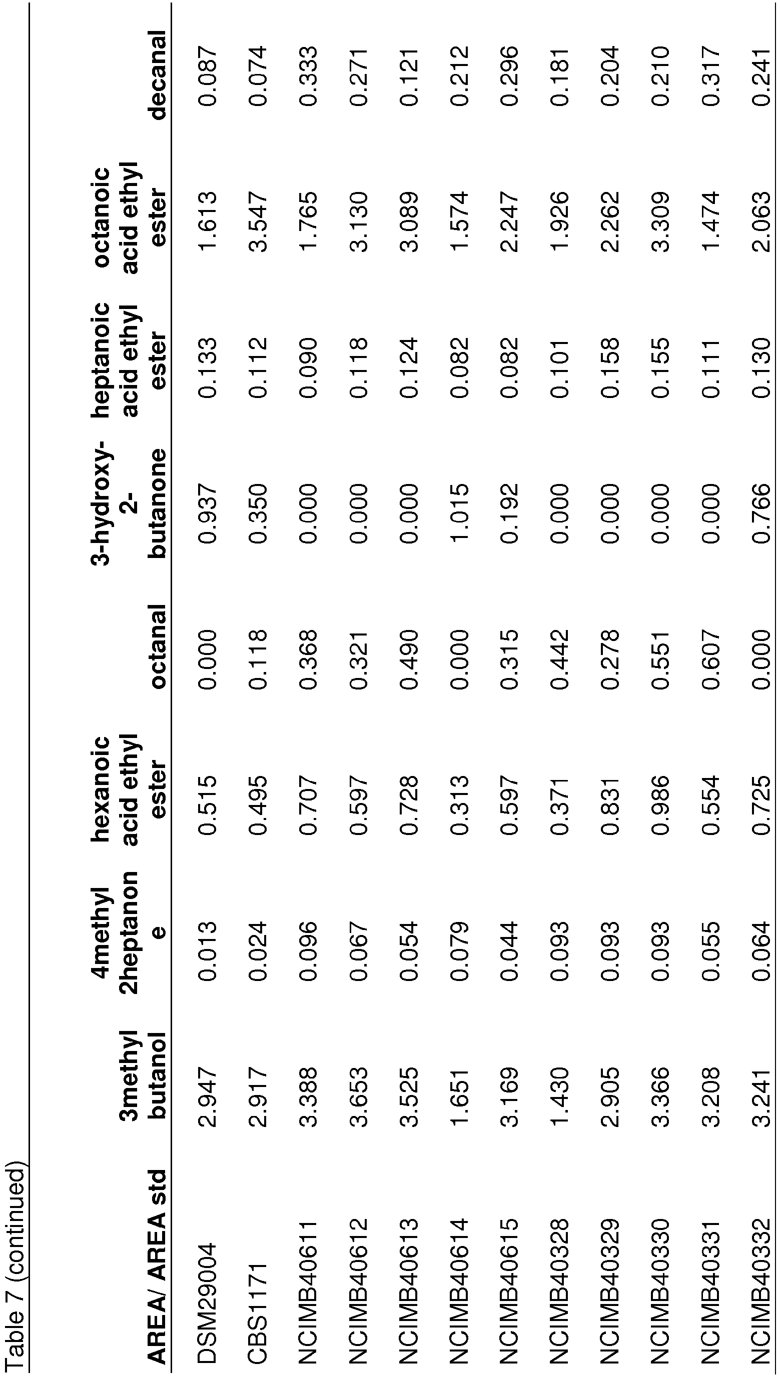

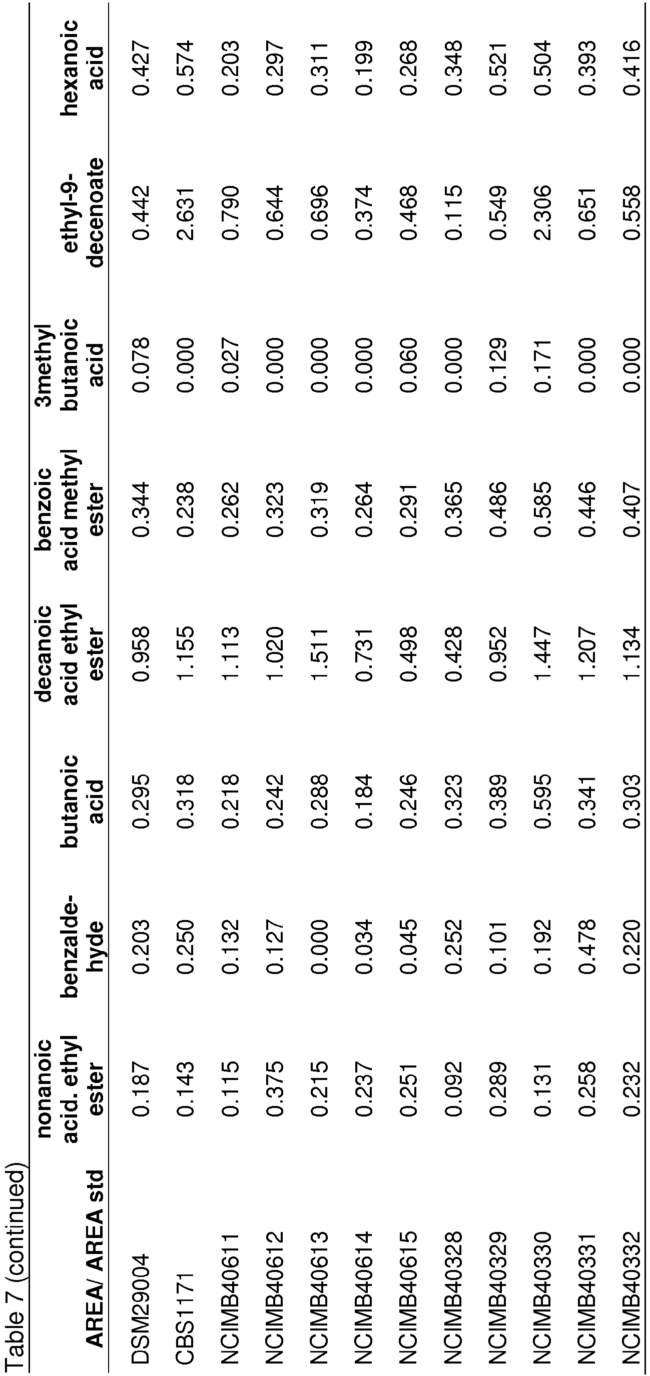

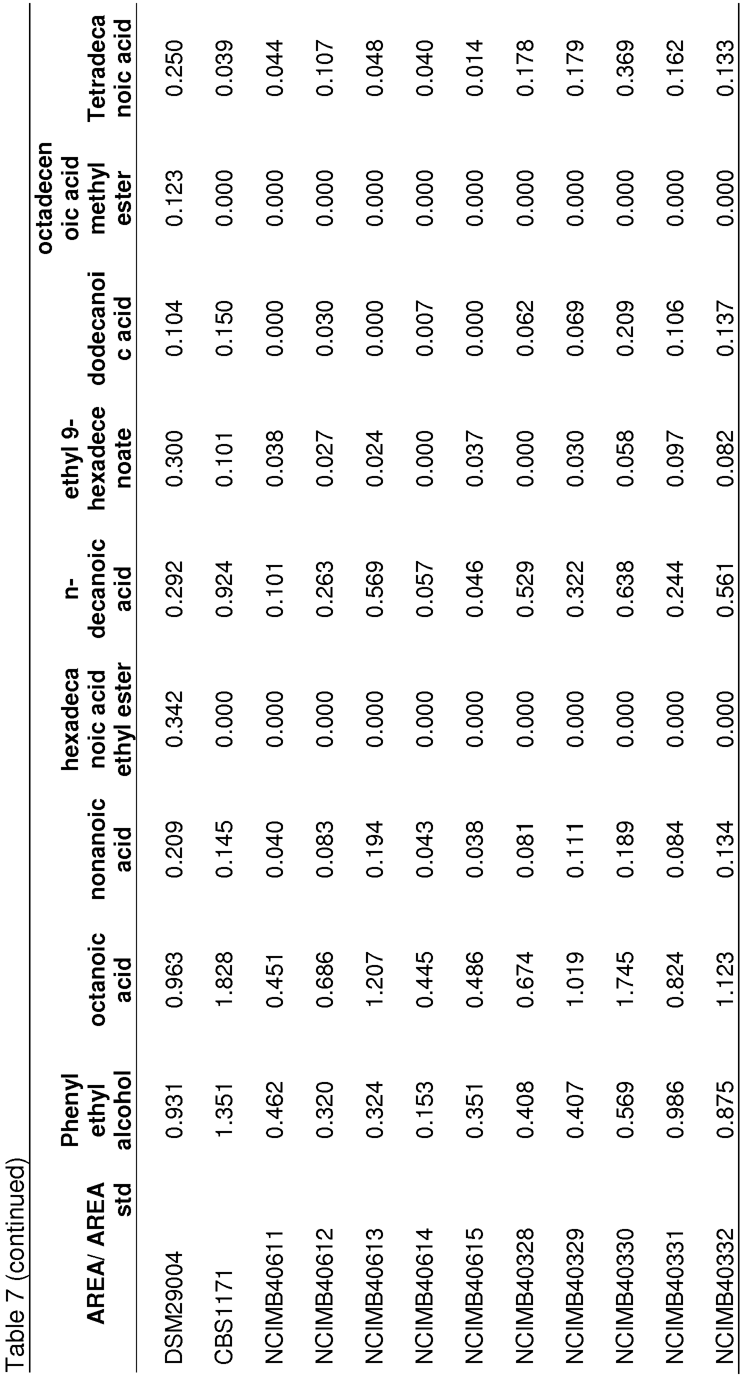

A Principal Component Analysis was then performed, in order to verify a further discrimination of the strains. It was observed that the first two components explain over 50% of the total variance and on the basis of the projection of the first two variables, the strains were placed on the projection graph as shown in Figure 5.

### Results:

Figure 5 shows that the NCIMB strains of the prior art are all grouped amongst each other (with the exception of NCIMB40330, which is in anyhow on the bottom (negative) part of the graph. Both strains CBS1171 ^{T} and DSM29004 are distant on this projection and are on the positive side of the y axis.

According to Figure 5, the positions of the strains on the graph is due mainly to the following variables: 3-hydroxy-2butanone, hexadecanoic acid ethyl ester, ethyl 9-hexadecanoate e octadecanoic acid methyl ester (Table 7).

The variables hexadecanoic acid ethyl ester and octadecanoic acid methyl ester seem to be produced only by the strain according to the present invention. In fact only the *S.cerevisiae* strain DSM29004 produces the volatile compounds: hexadecanoic acid ethyl ester and octadecanoic acid methyl ester.

These compounds are characterized by a fruity and smooth aroma.

The presence of these compounds makes the DSM 29004, grown on molasses-based medium, release a more pleasant aroma than the usual yeast strains grown on the same medium.

### Example 10: volatile compounds analysis released by the Saccharomyces cerevisiae DSM 29004 strain, in Medium 4 (M4) by SPME GC-MS.

The analysis was performed as explained in Example 9, inoculating the strain DSM 29004 in Medium 4 (M4) instead of MM. The composition of M4, that is based on barley malt extract and wheat malt extract, is described in Table 8.

**Table 8 Medium 4 (M4), made of barley malt and grain malt for batch culture;**

| INGREDIENTS: | CONCENTRATION |
|---|---|
| BARLEY MALT EXTRACT | 55.0 g/l |
| WHEAT MALT EXTRACT | 35.0 g/l |
| (NH₄)₂HPO₄ | 3.5 g/l |
| K₂HPO₄ | 0.05 g/l |
| CaCl₂ x 2H₂O | 0.15 g/l |
| ZnSO₄ x 7H₂O | 15.0 mg/l |
| FeCl₃ x 6H₂O | 5.0 mg/l |
| MnSO₄ x 2H₂O | 0.5 mg/l |
| BIOTIN | 0.05 mg/l |
| CALCIUM PANTOTHENATE | 2.0 mg/l |
| INOSITOL | 300.0 mg/l |
| NICOTINIC ACID | 4.0 mg/l |
| THIAMINE-HCl | 0.7 mg/l |
| PYRIDOXINE-HCl | 0.7 mg/l |
| RIBOFLAVIN | 0.7 mg/l |
| FOLIC ACID | 0.2 mg/l |

The strain DSM 29004 was incubated for 72 hours in the M4 medium and it produced a typical profile of volatile compounds constituted by the thirty molecules reported in Table 9. The experiment was repeated twice and the data are expressed as the mean value of the ratio of each peak area and standard peak area. The chromatogram of one of the replicates is depicted in Figure 6.

**Table 9. Quantification of the thirty volatile compounds produced by DSM 29004 after the growth in M4 for 72 hours.**

| AREA/ AREA std | DSM 29004 |
|---|---|
| Ethyl Acetate | 0.530 |
| 3methyl butanal | 0.000 |
| Ethanol | 18.039 |
| 1 propanol | 0.175 |
| 2-methyl 1-propanol | 0.286 |
| 3methyl, 1butanol acetate | 0.566 |
| 2methyl 2 heptanol | 0.166 |
| 3-methyl 1 butanol | 3.067 |
| 4-methyl 2-heptanone | 0.102 |
| hexanoic acid ethyl ester | 0.335 |
| Octanal | 0.157 |
| 3hydroxy-2butanone | 0.090 |
| heptanoic acid ethyl ester | 0.081 |
| Nonanal | 0.219 |
| octanoic acid ethyl ester | 2.412 |
| nonanoic acid, ethyl ester | 0.516 |
| benzaldehyde | 0.034 |
| decanoic acid ethyl ester | 0.680 |
| undecanoic acid ethyl ester | 0.209 |
| benzenacetaldehyde | 0.878 |
| ethyl-9-decenoate | 0.271 |
| acetic acid 2phenylethyl ester | 0.194 |
| hexanoic acid | 0.101 |
| dodecanoic acid ethyl ester | 0.000 |
| phenylethyl alcohol | 4.080 |
| octanoic acid | 0.737 |
| nonanoic acid | 0.035 |
| n-decanoic acid | 0.106 |
| ethyl 9-hexadecenoate | 0.506 |

By comparing Table 9 and Table 7 we can highlight the differences between the aroma profiles of DSM 29004 grown on M4 and MM media. The most relevant differences are evidenced in ethanol concentration, higher in M4, due to the higher sugar concentration, which favors the fermentative pathway. However, the concentration of some molecules with a relevant impact on the overall flavor is higher following the growth of DSM 29004 on M4. In fact, the following compounds resulted in higher proportion in M4: octanoic acid ethyl ester (2.41 vs. 1.61), phenylethyl alcohol (4.08 vs. 0.93), acetic acid phenylethyl ester (0.19 vs. 0), benzeneacetaldehyde (0.88 vs. 0), 4-methyl-2-heptanone (0.10 vs. 0.01), octanal (0.15 vs. 0), nonanoic acid ethyl ester (0.52 vs. 0.19). All these compounds have a strong impact on the flavor, enhancing the fruity and floral (rose-like) components of the flavor and reducing the stinging components of the flavor, which are annoying for the human taste. The stinging components of the flavor, instead, are typical of the fermentation of yeast grown on Molasses-based medium.

### Example 11: method to induce enzyme expression in DSM 29004 for metabolizing maltose and malt extract related compounds.

Three samples of total RNA are purified by three cultures of *S. cerevisiae* DSM 29004 grown for 18 hours at 27°C in YPD (Table 5), MM (Table 6) and M4 (Table 8) through ZR Fungal/Bacterial RNA MiniPrep (ZymoResearch). High quality total RNA (RIN value >8.0) is processed. The mRNA is first purified by polyA capture, while for bacterial sample the total RNA is first subjected to rRNA depletion. The purified mRNA is fragmented and converted to double-stranded cDNA. DNA adapters with sample-specific barcodes are ligated and a PCR amplification is performed. The "dUTP method" is used to generate strand-specific mRNA-seq libraries including barcoding and that are compatible with either single-read or paired-end sequencing. The Illumina TruSeq stranded RNA-seq library preparation kit is used. The library is size-selected using magnetic beads, resulting on average in libraries with insert size in the approximate range of 100-400 bp.

The sequence reads are additionally filtered and trimmed based on Phred quality scores. The filtered/trimmed reads are aligned against the reference sequence of the strain *S. cerevisiae* S288c using the CLCbio "RNA-Seq" software. Also (normalized) expression values are calculated and compared between the samples. Quality control is performed through boxplot analysis, principle component analysis (PCA) and hierarchical clustering of the normalized expression values.

The data shows in the sample of S. *cerevisiae* DSM 29004 grown in M4 when compared both with YPD and MM media a modulated expression of the genes of the glucosidase family genes and the genes related to the maltose metabolism, in particular the genes *mal11*, *mal11.3*, *mal11.5*, *mal12, mal13*, *mal13.3*, *mal13.5*, *mal31,* mal31.3, *mal31.5*, *mal32*, *mal33*, *mal33.3*, *mal33.5.*

These data show that the M4 medium enhances the expression of the genes related with dough fermentation in *S. cerevisiae* DSM 29004, since maltose is the sugar mainly present in baked products dough. Thus the DSM 29004 strain, grown on a M4 medium, and once in a baked product dough, will use the maltose more efficiently and will display more promptness, more quickness and more vigour, than the one grown on molasses-based medium, thus favouring the dough leavening. This characteristic of better leavening of the strain DSM 29004 grown on M4 medium represents a consistent advantage in the use of Lti strains, since it is known that these strains show, even at temperatures that are usually optimal for *S. cerevisiae* fermentation, a lower metabolic activity, and this usually results in an insufficient leavening or a too long time needed for decent leavening.

### Example 12: low sulfur dioxide production in the medium according to the invention by the strain DSM 29004 and its relevance for the interaction with lactic acid bacteria

The M4 barley malt extract and wheat malt extract based medium is prepared by sterilizing in autoclave at 121 °C for 15 minutes a solution of barley malt extract 55 g/l and wheat malt extract 35 g/l, then adding the other components sterilized in the following way: in autoclave, in the case of salts and by filtration in the case of vitamins.

A mid-exponential culture in YPD of the strain *Saccharomyces cerevisiae* DSM 29004, is normalized to a concentration of 0.1 OD (wavelength of 600 nm) and 2 ml is inoculated in 100 ml of the medium and grown at 27°C in a shaker for 36 hours. The strain *Saccharomyces cerevisiae* DSM 29004 shows a production of sulphur dioxide lower than 10 mg/l, that is compatible with the use of the discarded medium as a supplement for enhancing the growth of lactic acid bacteria in a sourdough. In fact sulphur dioxide, is usually produced by yeast during fermentation. The presence of sulphur dioxide inhibits the growth of lactic acid bacteria, thus is not advantageous in dough fermentation (Example 11) and would represent an insurmountable obstacle in the case the discarded medium was added to the dough together with the yeast strain as a supplement. Instead the medium discarded after the growth of DSM29004, not showing presence of sulphur dioxide, is suitable for being added to a dough as a supplement, thus exploiting its aforementioned capacity of enhancing the growth of lactic acid bacteria.

### Example 13: high alpha-aminic nitrogen concentrations available in the medium according to the invention by the strain DSM 29004 and its relevance for the interaction with lactic acid bacteria

The M4 barley malt extract and wheat malt extract based medium is prepared by sterilizing in autoclave at 121 °C for 15 minutes a solution of barley malt extract 55 g/l and wheat malt extract 35 g/l, then adding the other components sterilized in the following way: in autoclave, in the case of salts and by filtration in the case of vitamins.

A mid-exponential culture in YPD of the strains *Saccharomyces cerevisiae* DSM 29004, *S. cerevisiae* NCIMB 40611; *Saccharomyces cerevisiae* NCIMB 40612; *Saccharomyces cerevisiae* NCIMB 40613; *Saccharomyces cerevisiae* NCIMB 40614; *Saccharomyces cerevisiae* NCIMB 40615; *Saccharomyces cerevisiae* NCIMB 40328; *Saccharomyces cerevisiae* NCIMB 40329; *Saccharomyces cerevisiae* NCIMB 40330; *Saccharomyces cerevisiae* NCIMB 40331; *Saccharomyces cerevisiae* NCIMB 40332 *Saccharomyces cerevisiae* CBS1171^{T}, are normalized as described in Example 12 and inoculated in 100 ml of the medium, then grown at 27°C in a shaker for 36 hours.

The discarded medium at the end of the fermentation in the case of the strain DSM 29004 shows a remarkable concentration of alpha-aminic nitrogen of 465 mg/l (Table 10), while the average of all the other tested strains is 133 mg/l. This 3 folds-higher concentration of alpha-aminic nitrogen confers to the discarded medium an added value as supplement for the growth of lactic acid bacteria. In fact alpha-aminic nitrogen is a source of essential factors for the growth of microorganisms, such as lactic acid bacteria, especially the lactic acid bacteria that are well adapted to dough and sourdough. The presence of lactic acid bacteria is essential to improve dough texture and flavours, giving it a more natural fragrance (see Example 14).

**Table 10. Concentration of the alpha-aminic nitrogen in the discarded medium obtained after the growth of DSM 29004 and the yeast strains reported in the prior art.**

| STRAIN GROWN IN M4 MEDIUM | ALPHA-AMINIC NITROGEN (mg/l) |
|---|---|
| NCIMB 40328 | 148 |
| NCIMB 40329 | 108 |
| NCIMB 40330 | <150 |
| NCIMB 40331 | 109 |
| NCIMB 40332 | 112 |
| NCIMB 40611 | <150 |
| NCIMB 40612 | 123 |
| NCIMB 40613 | 143 |
| NCIMB 40614 | 18 |
| NCIMB 40615 | 125 |
| CBS 1171 | 110 |
| DSM 29004 | 465 |

### Example 14: effect of fresh yeast extract of DSM 29004 on Lactobacillus sanfranciscensis and comparison of the effect of the Medium 4 discarded at the end of the strain DSM 29004 and CBS 1171^{T} production on lactic acid bacteria

The *Lactobacillus sanfranciscensis* is known to require fresh yeast extract as a supplement in the medium DSM 225 (known also as "Dough Like Medium") produced according the protocol released by DSMZ (Table 11). The *Lactobacillus sanfranciscensis* is one of the most used bacteria for preparation of sourdoughs, because of its capacity to give the dough sour flavors typical of old-fashion handmade doughs. A fresh yeast extract from the strain DSM 29004 was prepared and used to grow Lactobacillus sanfranciscensis LMG17498. The growth was well promoted by the fresh DSM 29004 extract (Fig. 7a).

**Table 11. Composition of the medium DSM 225, also known as "Dough Like Medium".**

| INGREDIENTS: | CONCENTRATION |
|---|---|
| MALTOSE | 20 g/l |
| YEAST EXTRACT | 3.0 g/l |
| FRESH YEAST EXTRACT | 15 ml/l |
| TWEEN 80 | 0.3 g/l |
| CASEIN PEPTONE | 6.0 g/l |

The potentiality of the discarded M4 medium recovered after the harvest of the yeast cells of the strain DSM 29004 as a possible supplement to enhance the growth of the lactic acid bacteria in a sourdough was assessed through the analysis of the kinetic of growth in liquid MRS medium (Oxoid). The MRS medium was prepared according the instruction of the producer, except for the use of 9/10 of water to dissolve the powder. The solution was sterilized in autoclave, then in each bottle/vial was added 1/10 volume of the discarded M4 medium in which the strain DSM 29004 and CBS 1171^{T} were grown. The discarded media were added by filtration with membranes characterized by ore size of 0.45 µm. The kinetics of growth was monitored by measuring the Optical Density of the tube at 600 nm.

In the assay the strains *Lactobacillus brevis* DSM 20054^{T}, *Lactobacillus fermentum* LMG 6902^{T}, *Lactobacillus plantarum* DSM 20174^{T}, *Leuconostoc mesenteroides* LMG 6893^{T}, *Pediococcus pentosaceus* LMG 11488^{T} and *Pediococcus acidilactici* were used. The data for all the mentioned species are reported in Figure 7.

More in detail a mid-exponential culture in MRS of each strains was normalized to a concentration of 0.1 OD at 600 nm wavelength and 10 µl were inoculated in MRS as negative control, in MRS supplemented with the discarded M4 medium from DSM 29004 culture (MRS + M4 DSM29004) and with discarded M4 medium from CBS1171 ^{T} (MRS + M4 CBS1171). The kinetics of growth of these bacterial cultures was monitored for 48 hours by measuring the cell concentration with the spectrophotometer at 600 nm wavelength (Figures 7b, 7c, 7d, 7e, 7f, 7g).

Quality of cereal-based products is influenced by the functions of several ingredients, such as sourdough.

Sourdough is a mixture of flour and water that is fermented by a microflora composed of stable associations of yeast and lactic acid bacteria (LAB).

These LAB are mainly heterofermentative strains, elaborating lactic acid and acetic acid in the mixture, and hence resulting in a sour taste of the end product. Sourdough plays an important role in the preparation of bread dough to favour technological properties (for instance improved dough machinability), nutritional properties (for instance through phytate hydrolysis), organoleptic properties (for instance bread volume, crumb texture, and a unique flavour), and keeping properties (shelf-life).

LAB and yeasts are often associated in sourdough. The LAB : yeast ratio in sourdoughs is generally 100:1. Whereas in the majority of fermented foods homofermentative LAB play an important role, heterofermentative LAB are dominating in sourdough, especially when traditionally prepared. Indeed, acetic acid, an important end product of heterofermentation, plays a major role in the flavour of sourdough.

LAB are an important group of industrial starter cultures, applied in the production of fermented foods.

LAB contribute to the improvement of the taste properties of fermented foods, as well as to their biopreservatives and microbial safety.

The metabolic activity of LAB during fermentation improve dough property, texture and flavor; retard the stalling process and prevent leavened products from mould and bacteria spoilage. LAB contribute to nutritional value and healthiness of products.

The importance of the synergistic interactions between lactobacilli and yeast are based on the metabolism of carbohydrates and amino acids and the production of carbon dioxide.

They mainly produce L-lactic acid and acetic acid with lesser amount of other acids such as citric acid and malic acids.

Many rheological properties of dough rely on the metabolic activities of its resident LAB: lactic fermentation, acetic fermentation, proteolysis, synthesis of volatile compounds, anti-mould activity and antiropiness.

The metabolic activities of LAB fermentation improve dough properties, bread texture and flavor and retard the stalling process of bread.

The most important effect of LAB fermentation in dough have been considered for long time the decrease of pH, which have been caused with organic acid production. This drop in pH influences the viscoelastic behavior of dough and this is necessary to maintain handling and machinability in industrial production.

The ratio between lactic and acetic acid is an important factor that might affect the aroma profile and structure of final product. Acetic acid is responsible for a shorter and harder gluten, while lactic acid can gradually account for a more elastic gluten structure.

In addition, the activity of proteolytic and amylolytic enzyme present may be influenced to a greater degree by the pH profile of the biological acidification fermentation period in contrast to the rather instantaneous nature of the chemically acidified regime. Optimum activity of these enzymes, which play significant role in changes of dough constituents, achieve optimum activity at pH 4-5 for the proteolytic and pH 3.6 - 6.2 for the amylolytic enzymes. Other enzymes that might affect the structural components of the dough the activity of which is pH dependent include peroxidases, catalases, lipoxigenases and polyphenol oxydases.

It was reported that certain lactic acid bacteria are also able to produce exopolysacharides (EPS), which might have a positive effect on bread volume and shelf life. The most frequent EPS produced during sourdough fermentation are: fructans (levan, inulin) and glucans (reuteran, dextran, xanthan). EPS influence significantly to the texture improvement of dough. The production of EPS in sufficient amounts during dough fermentation would create the possibility to replace hydrocolloids in baking. Hydrocolloids have been reported to improve bread quality through stabilization of water-flour association in doughs matrix.

During dough fermentation LAB release also small peptides and free amino acids important for microbial growth and acidification of dough. Moreover small peptides and free amino acids are important as precursors for flavor development of the leavened baked products.

Sourdough LAB generally belongs to the genus *Lactobacillus, Leuconostoc*, *Pediococcus* or *Weisella.*

Kinetics of growth of *Lactobacillus spp.* in M4 show an higher grow of bacteria belong to *Pediococcus spp*., especially *P. pentosaceus* and *P.acidilactici* when MRS is supplied with discarded M4 from a DSM 29004 culture
*Pediococcus pentosaceus* are Gram-positive, facultatively anaerobic, non-motile and non-spore forming, members of the industrially important lactic acid bacteria. Like other lactic acid bacteria, *P. pentosaceus* are acid tolerant, cannot synthetize porphyrins, and possess a strictly fermentative metabolism with lactic acid as the major metabolic product.

Lactic acid is produced from hexose sugars via the Embden-Meyerhof pathway and from pentoses by the 6-phosphogluconate/phosphoketolase pathway. *P. pentosaceus* grow between pH 4.5 and 8.0, in 9-10% NaCl, hydrolyzes arginine and can utilize maltose.

Strains of *P. pentosaceus* have been reported to contain between three and five resident plasmids. Plasmid-linked traits include the ability to ferment raffinose, melibiose, and sucrose, as well as, the production of bacteriocins as antimicrobic agents.

*P. pentosaceus* has also a fungistatic effect due to the production of acetic acid.

*P. pentosaceus* is an homofermentative LAB and its fermentation results in high concentration of lactic acid, relative to acetic acid, resulting in a mild and flat sour taste very appreciate in dough.

There are two kinds of aromatic compounds that are produced during fermentation of dough. Non volatile compounds include organic acids produced by homo- and heterofermentative bacteria that acidify, decrease the pH, and produce aroma in the dough. Volatile compounds are composed of the alcohols, aldehydes, ketones, esters, and sulfur-containing compounds produced by biological and biochemical actions during fermentation and contribute flavor.

In particular, *P. pentosaceus* produces diacetyl and ethyl acetate that provide a pleasant flavor of butter, which gives a particular softness to the dough.

### BIBLIOGRAPHY:

Dutech C., Enjalbert J., Fournier E., Delmotte F., Barrès B., Carlier J., Tharreau D., Giraud T. (2007). Challenges of microsatellite isolation in fungi. Fungal Genet Biol. 44: 933-949.
Foury F., Roganti T., Lecrenier N., Purnelle B. (1998). The complete sequence of the mitochondrial genome of Saccharomyces cerevisiae. FEBS Lett. 440: 325-331.
Karim A.S., Curran K.A., Alper H.S. (2013). Characterization of plasmid burden and copy number in Saccharomyces cerevisiae for optimization of metabolic engineering applications. FEMS Yeast Res. 13: 107-116.
Legras J.L., Ruh O., Merdinoglu D., Karst F. (2005). Selection of hypervariable microsatellite loci for the characterization of Saccharomyces cerevisiae strains. Int J Food Microbiol. 102: 73-83.
Legras J.L., Merdinoglu D., Cornuet J.M., Karst F. (2007). Bread, beer and wine: Saccharomyces cerevisiae diversity reflects human history. Mol Ecol. 16: 2091-2102.
Montanari, C., Bargossi, E., Lanciotti, R., Chinnici, F., Gardini, F., Tabanelli, G. (2014). Effects of two different sourdoughs on the characteristics of Pandoro, a typical Italian sweet leavened baked good. LWT - Food Science and Technology 59: 289-299Ralser M., Kuhl H., Ralser M., Werber M., Lehrach H., Breitenbach M., Timmermann B. (2012). The Saccharomyces cerevisiae W303-K6001 cross-platform genome sequence: insights into ancestry and physiology of a laboratory mutt. Open Biol. 2:120093.
Richards K.D., Goddard M.R., Gardner R.C. (2009). A database of microsatellite genotypes for Saccharomyces cerevisiae. Antonie Van Leeuwenhoek. 96: 355-359.
Sturley S.L. & Young T. (1986). Genetic Manipulation of Commercial Yeast Strains. Biotechnol Genet Eng. 4: 1-38.
Thygesen J.B., Glerup H., Tarp B. (2012). Saccharomyces boulardii fungemia caused by treatment with a probioticum. BMJ Case Rep. 2012. pii: bcr0620114412.
Torriani S., Zapparoli G., Malacrinò P., Suzzi G., Dellaglio F. (2004). Rapid identification and differentiation of Saccharomyces cerevisiae, Saccharomyces bayanus and their hybrids by multiplex PCR. Lett Appl Microbiol. 38:239-244.
Vaudano E. & Garcia-Moruno E. (2008). Discrimination of Saccharomyces cerevisiae wine strains using microsatellite multiplex PCR and band pattern analysis. Food Microbiol. 25: 56-64.
Vaughan-Martini A. & Martini A. (2011). Chapter 61: Saccharomyces Meyen ex Rees'. In Kurtzman C.P., Fell J.W., Boekhout T., The Yeasts, a Taxonomic Study, 5th edition.

## Claims

1. A *Saccharomyces cerevisiae* yeast strain, which is deposited under deposit Accession No. DSM 29004 with International Deposit Authority Leibniz-Institut DSMZ - Deutsche Sammlung von Mikroorganismen und Zellkulturen GmbH.

2. The yeast strain according to claim 1, **characterized in that** said strain remains viable for at least 20 days at a temperature comprised between 0°C and +8°C, and that said strain successively shows fermentative vigour and growth activity at a temperature comprised between +12°C and +38°C, preferably between +25°C to +28°C.

3. The yeast strain according to claim 2, **characterized in that** said strain remains viable for at least 30 days at a temperature comprised between 0°C and +6°C, and that said strain successively shows fermentative vigour and growth activity at a temperature comprised between+12°C and +38°C, preferably between +25°C to +28°C.

4. The yeast strain according to anyone of claims 1 to 3, **characterized in that** said strain shows a greater fermentative vigour in dough leavening, when previously grown in a barley malt and wheat malt extract medium than when grown in a molasses-based medium, at a temperature comprised between +12°C and +38°C, preferably between +25°C to +28°C.

5. The yeast strain according to claim 4, wherein said barley malt and wheat malt extract medium is in a concentration ranging from 0.1 g/l to 100 g/l preferably comprised from 40 g/l to 80 g/l and said molasses-based medium has a glucose concentration ranging from 0.1 g/l to 200 g/l preferably from 20 g/l to 100 g/l.

6. A solid or fluid dough, comprising at least water, flour and yeast of the *Saccharomyces cerevisiae* strain according to anyone of claims 1 to 5.

7. The dough according to claim 6, wherein said yeast of the *Saccharomyces cerevisiae* strain is in the form of vital cells.

8. The dough according to anyone of claims 6 or 7, wherein said yeast of the *Saccharomyces cerevisiae* strain is in a mixture with other yeast and/or microorganisms.

9. The dough according to anyone of claims 6 to 8, **characterized in that**, when packaged in a sealed container at a temperature comprised between 0°C and +8°C for 20 days, or preferably at a temperature comprised between 0°C and +6°C for 30 days, does not present swellings in the container, development of abnormal smells or tastes or the formation of liquid inside the container.

10. Use of the strain according to anyone of claims from 1 to 5, for the production of solid or fluid dough.

11. A process for the preparation of refrigerated dough in a solid or fluid form, prepared by mixing at least water, flour, yeast of the *Saccharomyces cerevisiae* species, according to anyone of claims 1 to 5, wherein said dough is packaged in a container.

12. The process according to claim 11 wherein said dough is packaged in a sealed container having an internal modified atmosphere from 0 to 80% N₂ and from 0 a 60% CO₂, or in a sealed container having ordinary atmosphere packaging.

13. A process for the preparation of a medium having an alpha-amino nitrogen concentration higher than 300 mg/l, comprising the steps of:
a. growing the yeast strain according to anyone of claims 1 to 5 in a barley malt and wheat malt extract medium for at least 36 hours;
b. separating the yeast from the medium, preferably by filtration or by centrifugation;
c. obtaining a medium having an alpha-amino nitrogen concentration higher than 300 mg/l.

## Patentansprüche

1. *Saccharomyces cerevisiae*-Hefestamm, der unter der Hinterlegungs-Zugangsnummer DSM 29004 bei der internationalen Hinterlegungsstelle Leibniz-Institut DSMZ- Deutsche Sammlung von Mikroorganismen und Zellkulturen GmbH hinterlegt ist.

2. Hefestamm gemäß Anspruch 1, **dadurch gekennzeichnet, dass** der Stamm für wenigstens 20 Tage bei einer Temperatur zwischen 0 °C und +8 °C lebensfähig bleibt und dass der Stamm sukzessive fermentative Kraft und Wachstumsaktivität bei einer Temperatur zwischen +12 °C und +38 °C, vorzugsweise zwischen +25 °C und +28 °C zeigt.

3. Hefestamm gemäß Anspruch 2, **dadurch gekennzeichnet, dass** der Stamm für wenigstens 30 Tage bei einer Temperatur zwischen 0 °C und +6 °C lebensfähig bleibt und dass der Stamm sukzessive fermentative Kraft und Wachstumsaktivität bei einer Temperatur zwischen +12 °C und +38 °C, vorzugsweise zwischen +25 °C und +28 °C zeigt.

4. Hefestamm gemäß einem der Ansprüche 1 bis 3, **dadurch gekennzeichnet, dass** der Stamm eine größere fermentative Kraft beim Teigaufgehen zeigt, wenn er vorher bei einer Temperatur zwischen +12 °C und +38 °C, vorzugsweise zwischen +25 °C bis +28 °C in einem Gerstenmalz- und Weizenmalzextraktmedium gewachsen ist als wenn er in einem Melassebasierten Medium gewachsen ist.

5. Hefestamm gemäß Anspruch 4, wobei das Gerstenmalz- und Weizenmalzextraktmedium in einer Konzentration im Bereich von 0,1 g/l bis 100 g/l, vorzugsweise von 40 g/l bis 80 g/l, ist, und das Melasse-basierte Medium eine Glucosekonzentration im Bereich von 0,1 g/l bis 200 g/l, vorzugsweise von 20 g/l bis 100 g/l, hat.

6. Fester oder flüssiger Teig, der wenigstens Wasser, Mehl und Hefe des *Saccharomyces cerevisiae-*Stamms gemäß einem der Ansprüche 1 bis 5 umfasst.

7. Teig gemäß Anspruch 6, wobei die Hefe des *Saccharomyces cerevisiae-*Stamms in der Form vitaler Zellen ist.

8. Teig gemäß einem der Ansprüche 6 oder 7, wobei die Hefe des *Saccharomyces cerevisiae*-Stamms in einem Gemisch mit anderer Hefe und/oder Mikroorganismen ist.

9. Teig gemäß einem der Ansprüche 6 bis 8, **dadurch gekennzeichnet, dass** er, wenn er in einem dicht geschlossenen Behälter verpackt ist, bei einer Temperatur zwischen 0 °C und +8 °C für 20 Tage und vorzugsweise bei einer Temperatur zwischen 0 °C und +6 °C für 30 Tage kein Aufquellen im Behälter, keine Entwicklung von abnormalen Gerüchen oder abnormalen Geschmacksrichtungen oder keine Bildung von Flüssigkeit im Inneren des Behälters zeigt.

10. Verwendung des Stamms gemäß einem der Ansprüche 1 bis 5 für die Herstellung von festem oder flüssigem Teig.

11. Verfahren zur Herstellung von gekühltem Teig in einer festen oder flüssigen Form, der durch Mischen von wenigstens Wasser, Mehl, Hefe der *Saccharomyces cerevisiae*-Spezies gemäß einem der Ansprüche 1 bis 5 hergestellt wird, wobei der Teig in einem Behälter verpackt wird.

12. Verfahren gemäß Anspruch 11, wobei der Teig in einem dicht geschlossenen Behälter, der eine innere modifizierte Atmosphäre von 0 bis 80 % N₂ und von 0 bis 60 % CO₂ hat, oder in einem dicht geschlossenen Behälter, der eine Normalatmosphärenverpackung hat, verpackt wird.

13. Verfahren zur Herstellung eines Mediums, das eine alpha-Aminostickstoffkonzentration von höher als 300 mg/l hat, umfassend die Schritte:
a. Wachsen-lassen des Hefestamms gemäß einem der Ansprüche 1 bis 5 in einem Gerstenmalz- und Weizenmalzextraktmedium für wenigstens 36 Stunden;
b. Abtrennen der Hefe aus dem Medium, vorzugsweise durch Filtration oder durch Zentrifugation;
c. Erhalten eines Mediums, das eine alpha-Aminostickstoffkonzentration von höher als 300 mg/l hat.

## Revendications

1. Souche de levure *Saccharomyces cerevisiae,* déposée sous le numéro d'accession DSM 29004 auprès de l'autorité de dépôt internationale Leibniz-Institut DSMZ - Deutsche Sammlung von Mikroorganismen und Zellkulturen GmbH.

2. Souche de levure selon la revendication 1, **caractérisée en ce que** ladite souche reste viable pendant au moins 20 jours à une température comprise entre 0 °C et + 8 °C, et que ladite souche présente successivement une vigueur fermentative et une activité de croissance à une température comprise entre + 12 °C et + 38 °C, de préférence entre + 25 °C et + 28 °C.

3. Souche de levure selon la revendication 2, **caractérisée en ce que** ladite souche reste viable pendant au moins 30 jours à une température comprise entre 0 °C et +6 °C, et que ladite souche présente successivement une vigueur fermentative et une activité de croissance à une température comprise entre + 12 °C et + 38 °C, de préférence entre + 25 °C et + 28 °C.

4. Souche de levure selon l'une quelconque des revendications 1 à 3, **caractérisée en ce que** ladite souche présente une vigueur fermentative supérieure lors de la levée de la pâte lorsqu'elle est préalablement cultivée dans un milieu d'extrait de malt d'orge et de malt de blé que lorsqu'elle est cultivée dans un milieu à base de mélasse à une température comprise entre + 12 °C et + 38 °C, de préférence entre + 25 °C et + 28 °C.

5. Souche de levure selon la revendication 4, dans laquelle ledit milieu d'extrait de malt d'orge et de malt de blé est présent dans une concentration allant de 0,1 g/l à 100 g/l, de préférence comprise entre 40 g/l et 80 g/l et ledit milieu à base de mélasse a une concentration en glucose allant de 0,1 g/l à 200 g/l, de préférence de 20 g/l à 100 g/l.

6. Pâte solide ou liquide comprenant au moins de l'eau, de la farine et de la levure de la souche *Saccharomyces cerevisiae* selon l'une quelconque des revendications 1 à 5.

7. Pâte selon la revendication 6, dans laquelle ladite souche de levure de *Saccharomyces cerevisiae* est sous la forme de cellules vitales.

8. Pâte selon l'une quelconque des revendications 6 ou 7, dans laquelle ladite levure de la souche *Saccharomyces cerevisiae* est en mélange avec d'autres levures et/ou microorganismes.

9. Pâte selon l'une quelconque des revendications 6 à 8, **caractérisée en ce que**, lorsqu'elle est emballée dans un récipient scellé à une température comprise entre 0 °C et + 8 °C pendant 20 jours, ou de préférence à une température comprise entre 0 °C et + 6 °C pendant 30 jours, ne présente pas de gonflement dans le récipient, de développement d'odeurs ou de goûts anormaux ou de formation de liquide à l'intérieur du récipient.

10. Utilisation de la souche selon l'une quelconque des revendications 1 à 5, pour la production de pâte solide ou liquide.

11. Procédé de préparation de pâte réfrigérée sous forme solide ou liquide, préparé en mélangeant au moins de l'eau, de la farine, de la levure de l'espèce *Saccharomyces cerevisiae,* selon l'une quelconque des revendications 1 à 5, dans lequel ladite pâte est emballée dans un récipient.

12. Procédé selon la revendication 11, dans lequel ladite pâte est emballée dans un récipient scellé ayant une atmosphère interne modifiée de 0 à 80 % de N₂ et de 0 à 60 % de CO₂, ou dans un récipient scellé ayant un emballage à atmosphère ordinaire.

13. Procédé de préparation d'un milieu ayant une concentration d'alpha-amino azote supérieure à 300 mg/l, comprenant les étapes consistant à:
a. faire croître la souche de levure selon l'une quelconque des revendications 1 à 5 dans un milieu d'extrait de malt d'orge et de malt de blé pendant au moins 36 heures;
b. séparer la levure du milieu, de préférence par filtration ou par centrifugation;
c. obtenir un milieu ayant une concentration en alpha-amino azote supérieure à 300 mg/l.
